(19) Europäisches Patentamt / European Patent Office / Office européen des brevets

(11) EP 2 808 327 A1

(12) **EUROPEAN PATENT APPLICATION**

(43) Date of publication:
**03.12.2014 Bulletin 2014/49**

(51) Int Cl.:
***C07D 487/04*** *(2006.01)* ***H01L 51/00*** *(2006.01)*

(21) Application number: **13169825.0**

(22) Date of filing: **29.05.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Solvay SA**
**1120 Brussels (BE)**

(72) Inventors:
- **Caille, Jean-Raphael**
  **5000 NAMUR (BE)**
- **Dabeux, Francois**
  **1140 EVERE (BE)**
- **Bolsee, Jean-Christophe**
  **1120 BRUSSELS (BE)**
- **Fenoll, Mathieu**
  **1030 SCHAERBEEK (BE)**

(74) Representative: **Dr. Langfinger & Partner**
**In der Halde 24**
**67480 Edenkoben (DE)**

(54) **Novel compounds with semiconducting properties**

(57) Novel compounds of formula (1)

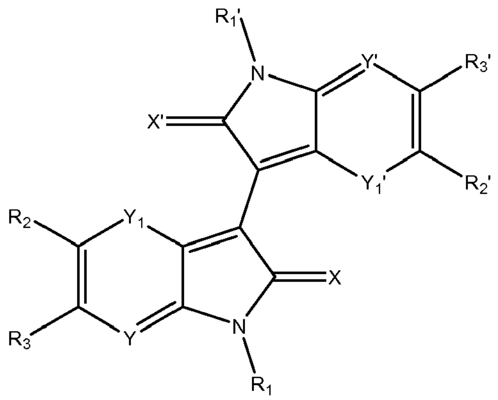

(1)

wherein X and X', independent of each other, represent O or S,
Y and Y', independent of each other represent N or P,
$Y_1$ and $Y_1$', independent of each other, represent -NH-, -O-, -S- or -PH-
$R_1$ and $R_{1'}$, independent of each other represent hydrogen, a $C_1$-$C_{30}$ hydrocarbyl or a $C_1$-$C_{30}$-heterohydrocarbyl group, $R_2$, $R_3$, $R_2$' and $R_3$', independent of each other represent a hydrogen, halogen, a $C_1$-$C_{20}$ hydrocarbyl or a $C_1$-$C_{20}$ heterohydrocarbyl group or $R_2$ and $R_3$ respectively $R_2$' and $R_3$' form together a substituted or unsubstituted five or six membered carbocyclic or heterocyclic, aromatic or heteroaromatic ring, which ring may be part of a fused ring system.

EP 2 808 327 A1

## Description

The present invention relates to novel compounds having semiconducting properties and to novel polymers derived from such compounds and the use of the compounds and the polymers in organic electronic devices.

Organic semiconductors are currently under intensive investigation and research as materials which may be used in organic electronic devices, such as transistors, OLEDs, RFID tags, display backplanes (e.g. for e-readers or similar devices) or the like.

Indigo and isoindigo derivatives have been recognized as efficient organic semiconductors when used as small molecules or as building blocks in polymeric or copolymeric structures.

Reynolds et al, Macromolecules 2010, 43, 8348-8352 discloses isoindigo-compounds and donor-acceptor conjugated polymers having repeating units based on the said isoindigo compounds. The compounds and polymers have the structural element

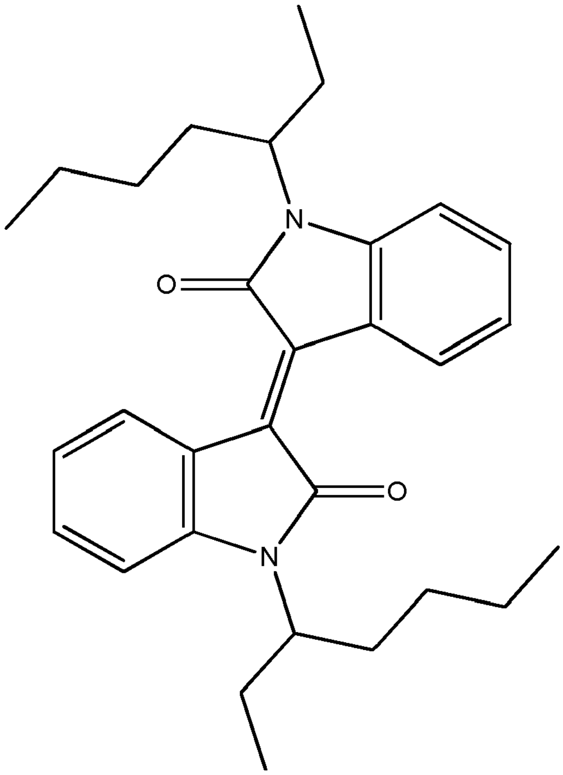

The compounds are said to be suitable for use in organic field effect transistors or photovoltaic devices.

Reynolds et al in Organic Letters 2010, Vol. 12 No.4, 660-663 describe the synthesis of isoindigo based oligothiophenes for molecular bulk heterojunction solar cells.

Reynolds et al., Macromolecules 2011, 44, 6303-6310 describe n-type conjugated polyisoindigos.

Pei et al., Adv. Mater. 2012, DOI: 10.1002/adma.201202689, describes the influence of alkyl chain branching positions on the hole mobilities of polymer thin film transistors comprising polymers based on isoindigo derivatives.

DE 1 196 205 relates to the synthesis of 4,7-diaza-2-oxinole derivatives of formula

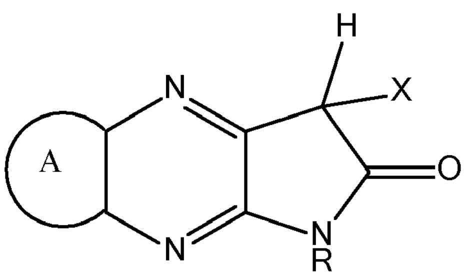

wherein A is an optionally substituted aryl ring and X is a hydrogen or a chlorine atom and R is a substituent.

Reicheneder et al., Angew. Chem. 81, 19 (1969), 742 discloses novel pigment dyes obtained from dichloromaleinimides which have the following structure

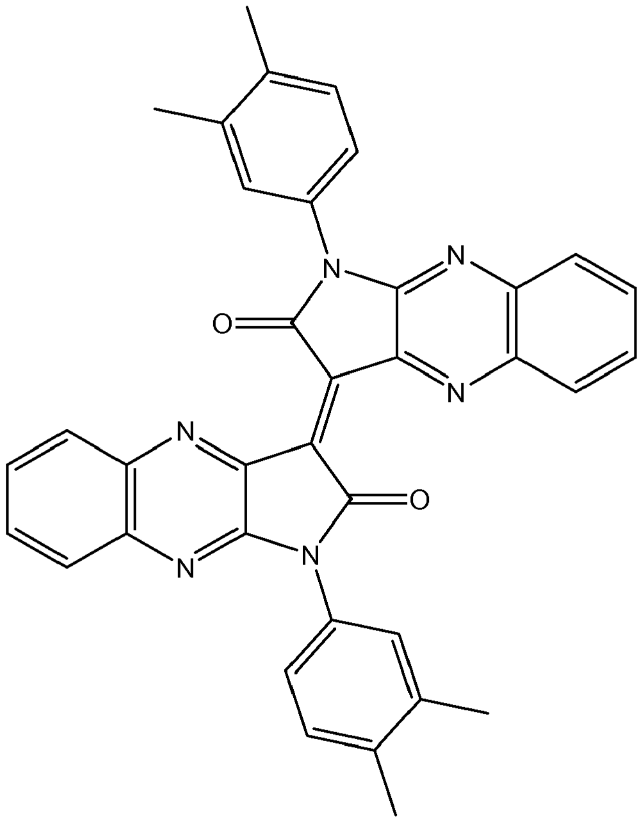

which are said to be intensely coloured dyes. Usability for any other purpose than dyeing is not disclosed.

The known organic semiconductors, in particular of the indigo or isoindigo type are not yet fully satisfactory in performance and thus there still exists a need for further organic semiconductors which are readily available and which have improved performance properties when used in organic electronic devices.

Accordingly it was an object of the present invention to provide novel organic semiconductors useful in organic electronic devices.

This object has been achieved with the compounds as defined in claim 1. Further aspects of the present invention relate to compounds as defined in claim 9 or to oligomers, homo- and copolymers in accordance with claim 8.

Still another aspect of the present invention relates to compositions of matter comprising the compounds as defined in claims 1 or 9 or oligomers and homo- or copolymers as defined in claim 8 and an electron acceptor compound.

Preferred embodiments of the present invention are set forth in the dependent claims and the detailed description hereinafter.

A first aspect of the present invention relates to compounds of formula (1)

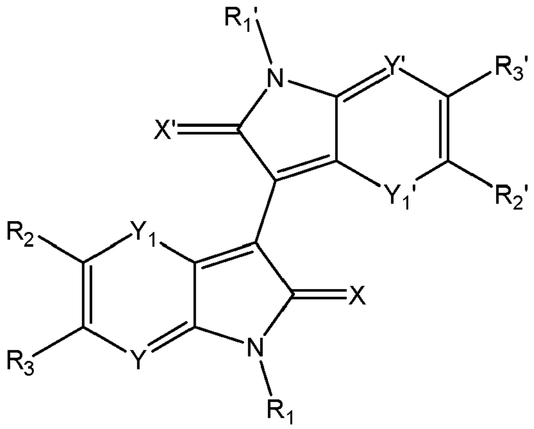

(1)

wherein X and X', independent of each other, represent O or S,
Y and Y', independent of each other represent N, or P,
$Y_1$ and $Y_1$', independent of each other, represent NH, O, S or PH
$R_1$ and $R_{1'}$, independent of each other represent hydrogen, a $C_1$-$C_{30}$ hydrocarbyl or a $C_1$-$C_{30}$-heterohydrocarbyl group,
$R_2$, $R_3$, $R_2$' and $R_3$', independent of each other represent hydrogen, halogen, a $C_1$-$C_{20}$ hydrocarbyl or a $C_1$-$C_{20}$ heterohydrocarbyl group or $R_2$ and $R_3$ respectively $R_2$' and $R_3$' form together a substituted or unsubstituted five or six membered carbocyclic or heterocyclic, aromatic or heteroaromatic ring, which ring may be part of a fused ring system.

According to a first preferred embodiment of the present invention, $R_2$ and $R_3$, and/or $R_2$' and $R_3$' form together a substituted or unsubstituted five or six membered aromatic or heteroaromatic ring, which ring may be part of a fused ring system.

The respective compounds are represented by formula (2)

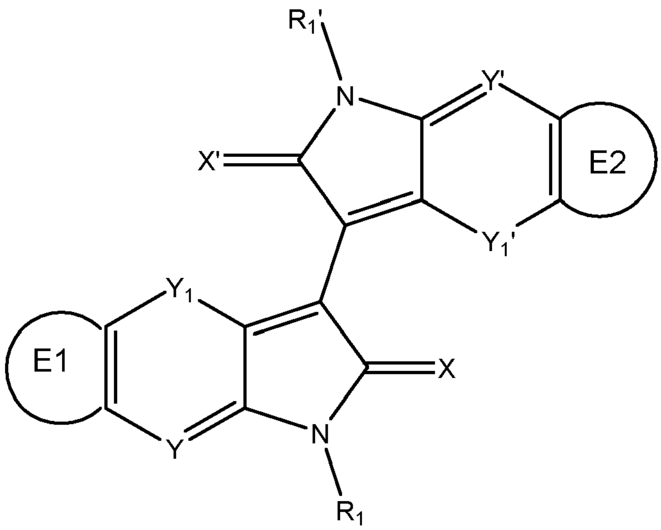

(2)

wherein E1 and E2 represent a substituted or unsubstituted five or six membered aromatic or heteroaromatic ring.

The five or six membered aromatic or heteroaromatic ring is preferably selected from the following ring systems

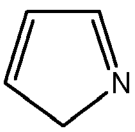 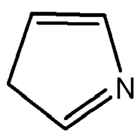 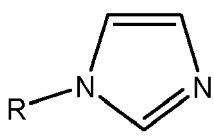 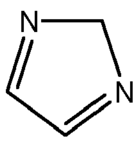 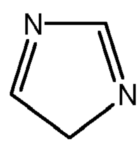

2 *H*-pyrrole 3*H*-pyrrole 1-substituted 1*H*imidazole 2H-imidazole 4*H*-imidazole

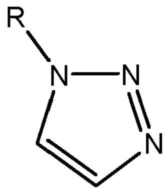

1-substituted-1*H*-1,2,3-triazole

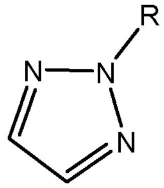

2-substituted-2H-1,2,3-triazole

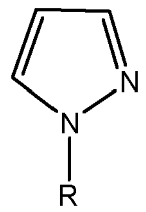

1-substituted-1 *H* -pyrazole

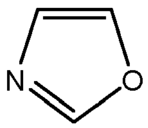

oxazole

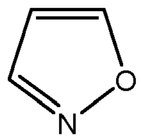

isoxazole

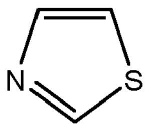

thiazole

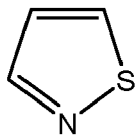

isothiazole

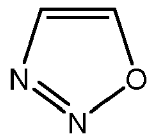

1,2,3-oxadiazole

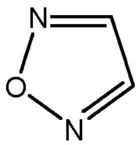

1,2,5-oxadiazole

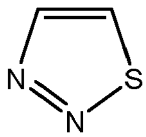

1,2,3-thiadiazole

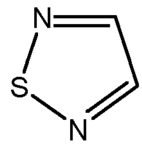

1,2,5-thiadiazole

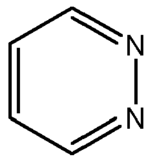

pyridazine

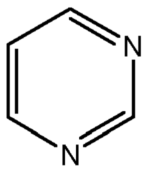

pyrimidine

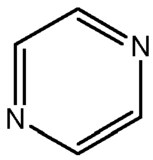

pyrazine

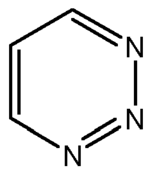

1,2,3-triazine

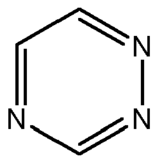

1,2,4-triazine

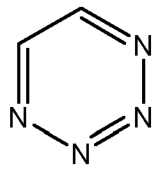

1,2,3,4-tetrazine

If $R_2$ and $R_3$ respectively $R_2$' and $R_3$' represent a ring system, such ring system may be substituted or unsubstituted.

Suitable substituents of respective ring systems are $C_1$-$C_{30}$ hydrocarbyl or $C_1$-$C_{30}$ heterohydrocarbyl groups, halogen or cyano groups.

If the compounds of formula (1) or (2), in accordance with a preferred embodiment of the present invention, are used as reagents for the synthesis of oligomers or homo- or co-polymers comprising moieties or repeating units or of compounds which comprise monovalent or divalent groups derived from compounds of formula (1), the substituents at the ring systems formed by $R_2$ and $R_3$ respectively $R_2$' and $R_3$' are preferably selected from halogen, $Sn(R_x)_3$ or boronic acid esters as functional groups which render the compounds of formulae (1) and (2) suitable for a variety of coupling reactions. Further details will be given hereinafter.

Preferably Y and Y' and $R_1$ and $R_1$' are identical.

$R_1$ and $R_1$' may be the same or different and preferably are identical and are preferably selected from $C_1$-$C_{30}$ linear or branched alkyl groups.

Preferred substituents $R_1$ and $R_1$' are

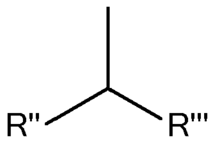

and

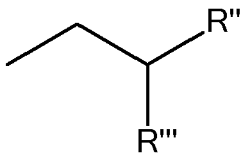

wherein R" and R"', which may be the same or different at each occurrence, are $C_1$-$C_{15}$ alkyl groups, preferably $C_1$ to $C_{12}$ alkyl groups, particularly preferred $C_1$ to $C_{10}$ alkyl groups, e.g. ethyl, butyl, pentyl, hexyl, octyl or decyl groups, which might be linear or branched, preferably linear.

Compounds wherein Y is identical to Y' and $Y_1$ is identical to $Y_1$' represent a preferred group of compounds in accordance with the present invention.

In accordance with another preferred embodiment, at least one of Y and Y' is, preferably both Y and Y' are N.

Furthermore, in accordance with another preferred embodiment, $Y_1$ and $Y_1$' represent NH.

Compounds with Y and Y' being N and $Y_1$ and $Y_1$' being NH are particularly preferred.

Compounds in which X and X' are identical represent another preferred embodiment and preferably at least one of X and X', preferably X and X' are O.

Furthermore, in accordance with still another preferred embodiment of the invention, X is identical to X' and $R_1$ and $R_1$' are identical.

Compounds (3) to (11) below are shown as a group of particularly preferred compounds of formula (1) respectively formula (2). As will be easily recognized, all these compounds conform to formula (1) and (2)

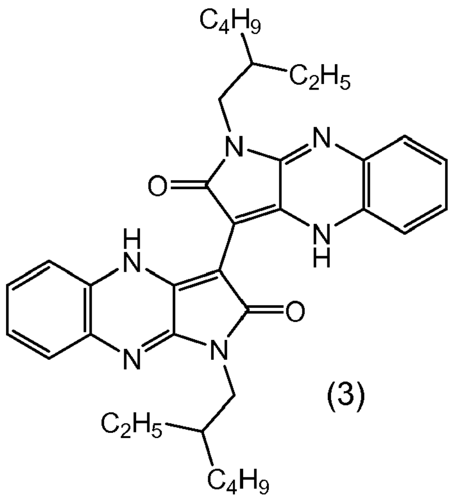

(3)

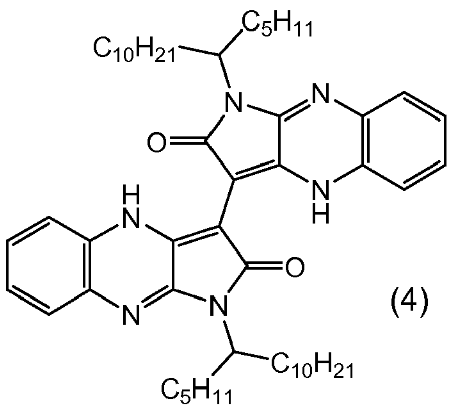

(4)

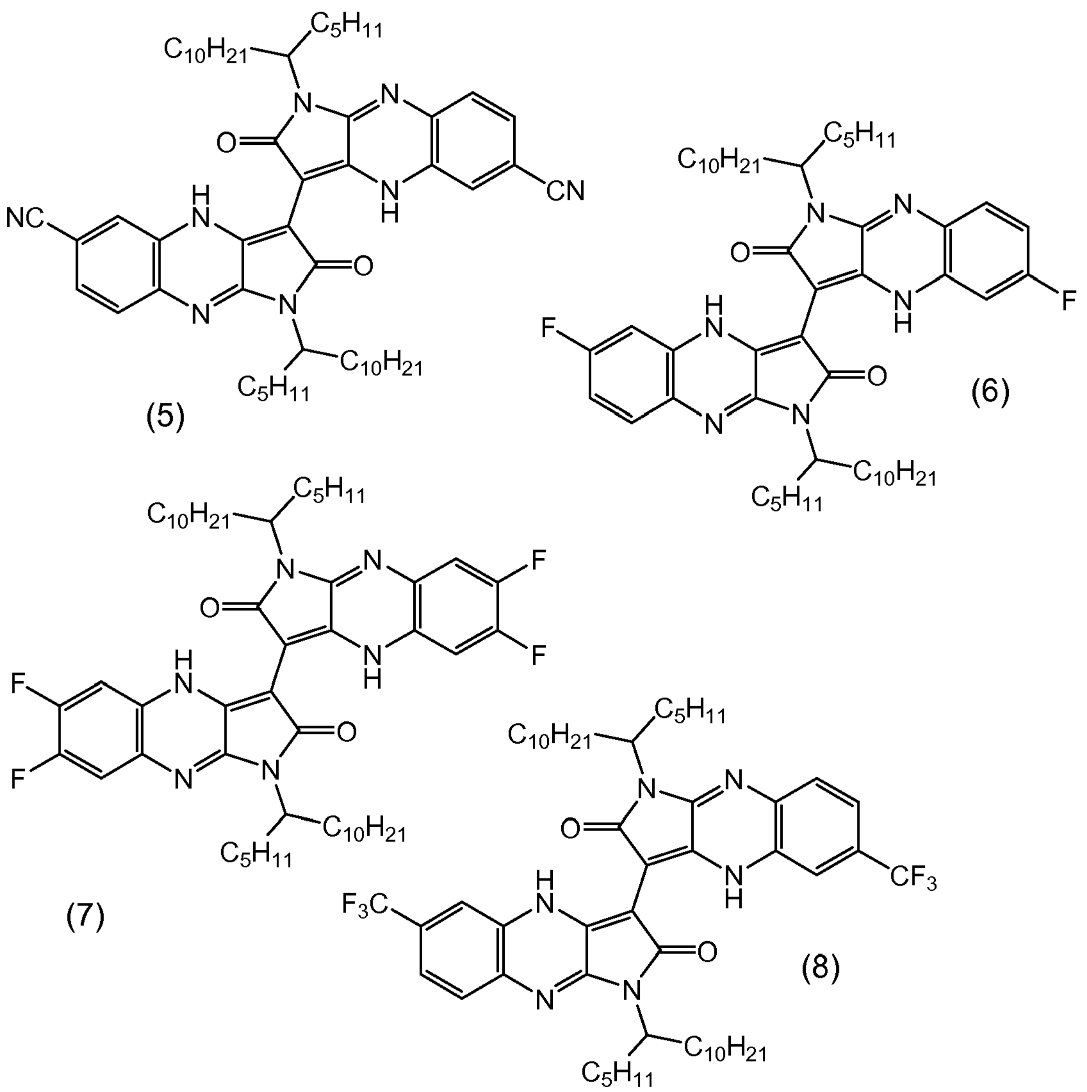

(5)

(6)

(7)

(8)

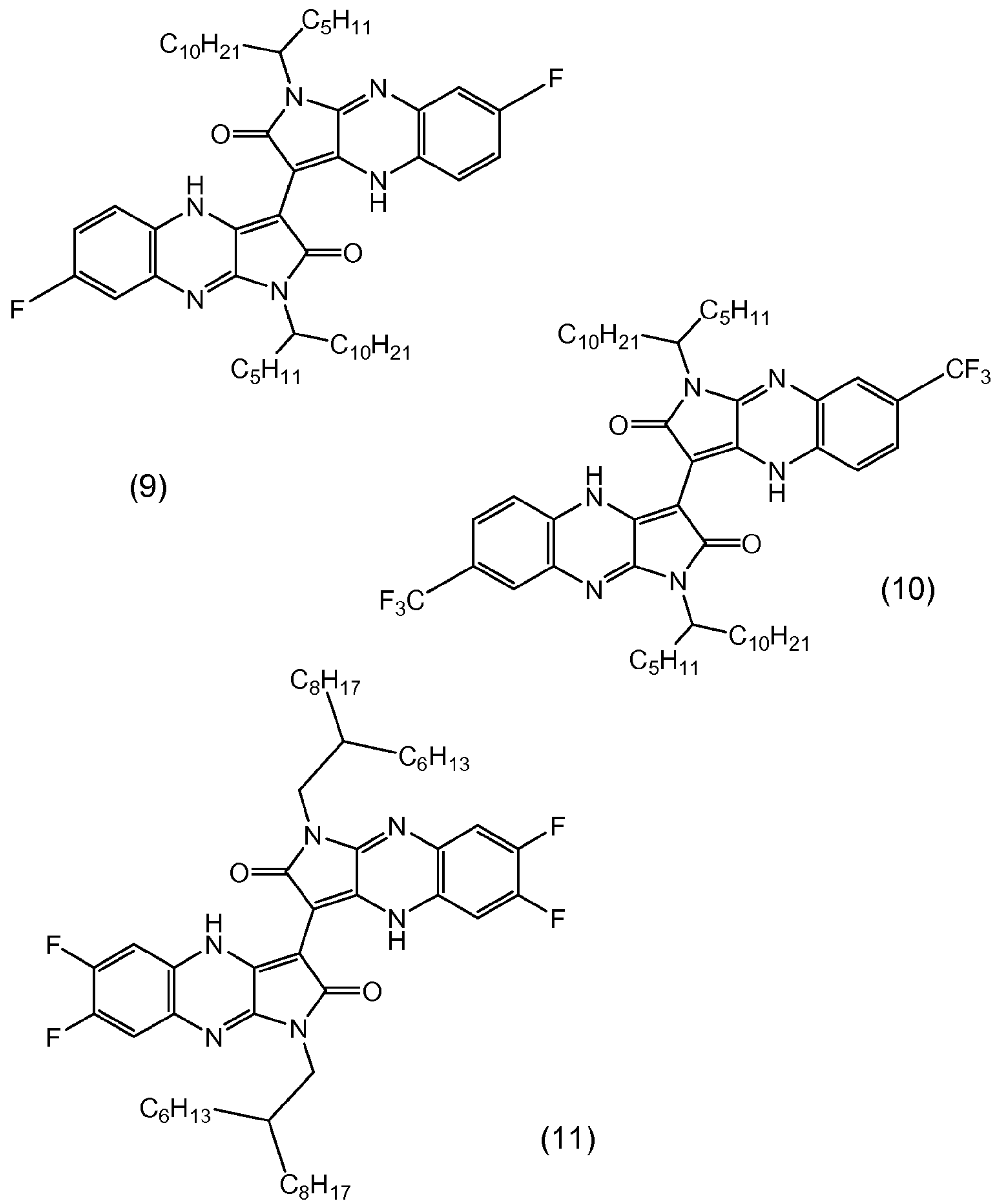

(9)

(10)

(11)

Certain compounds of formula (1) or (2) can occur in a number of different tautomeric forms and all the respective tautomers for the purpose of the present invention are within the scope of formula (1).

The following structures show examples of such tautomers

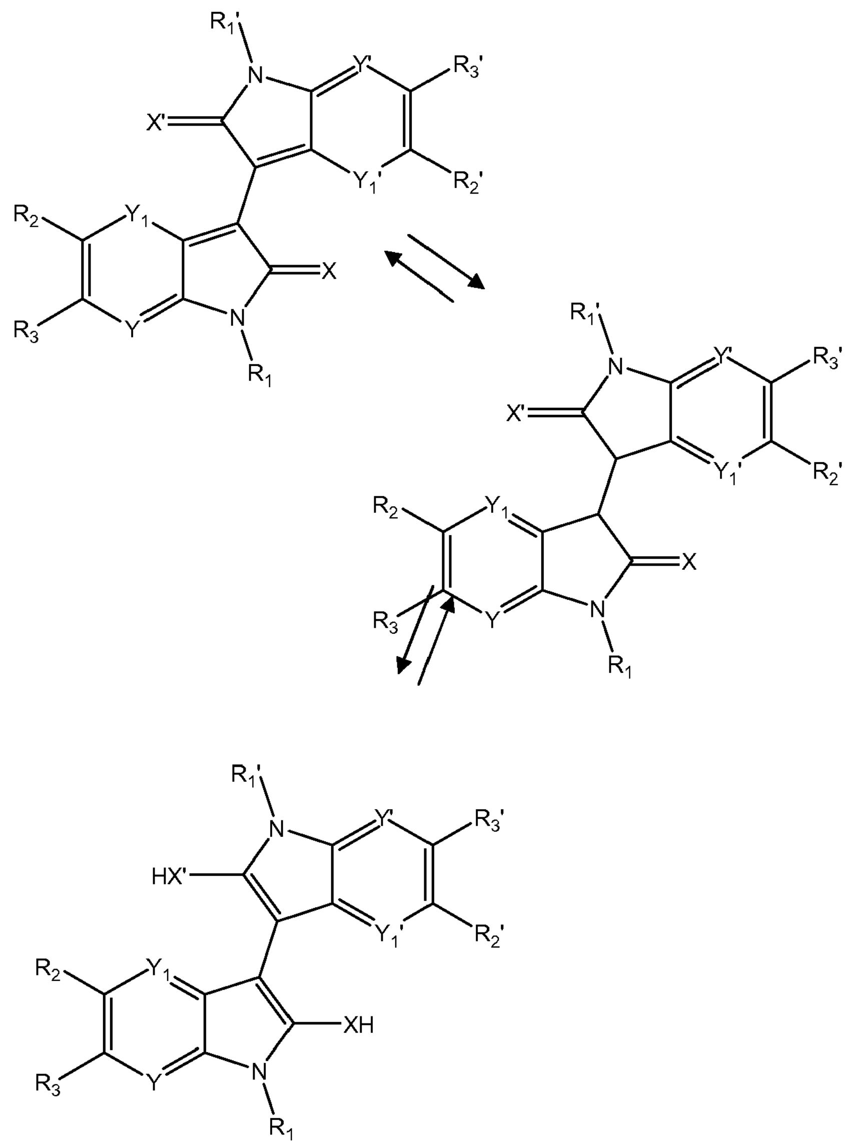

It is apparent to the skilled person that such tautomers require a certain combination of substituents Y, $Y_1$, Y' and $Y_1$', i.e. not all compounds of formula (1) or (2) can form tautomers as shown above.

The synthesis of compounds of formula (1) or (2) in accordance with the present invention is exemplified in a reaction scheme below for compounds wherein Y and Y' are N and $Y_1$ and $Y_1$' are NH. The skilled person, based on his professional knowledge, will modify the reaction conditions and the reagents to obtain compounds of formulae (1) and (2) with other combinations of substituents.

Step 1:

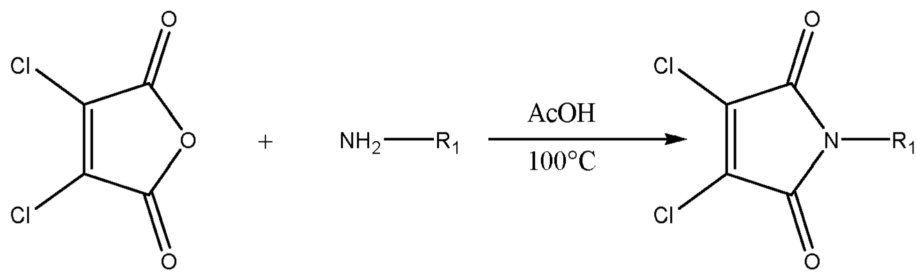


wherein $R_1$ is preferably an optionally substituted alkyl chain

Step 2:

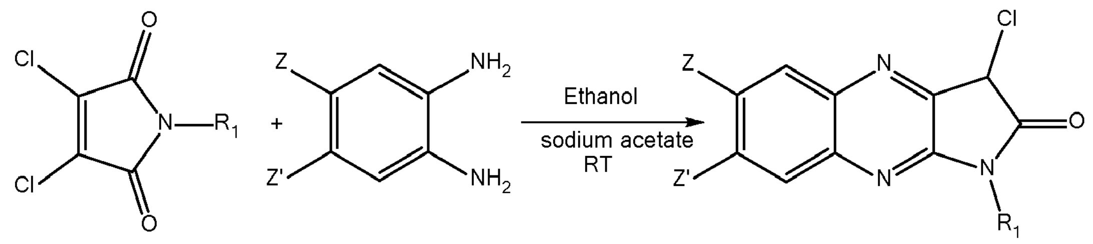


wherein Z and Z' are as defined below

Step 3

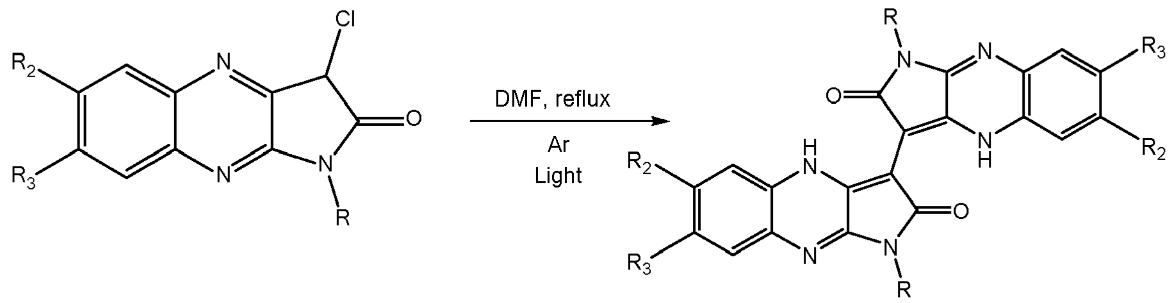


It is readily apparent to the skilled person how to modify the reaction conditions, the solvents and the starting materials to obtain specific desired compounds of formulae (1) or (2) so that further detailed information is not necessary here.

Suitable preferred diamines which may be used in step 2 of the reaction scheme depicted above may be represented by the general formula

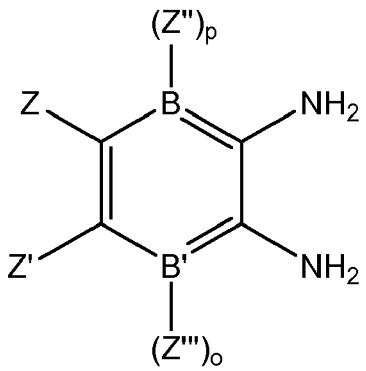

wherein

Z, Z', Z" and Z"', independent of one another, may be hydrogen, halogen, CN, COOH,-$COOR_a$, C(=O)-$R_a$, OH, $OR_a$, $C_1$-$C_8$ alkyl, $C_1$-$C_8$ haloalkyl, SCN, $NH_2$, $N(R_aR_b)$, $(O\text{-}CH_2\text{-}CH_2)_n$-OH, substituted or unsubstituted $C_5$-$C_{30}$ aryl, substituted $C_2$-$C_{20}$ heteroaryl, $C_2$-$C_{20}$ alkenyl, $C_2$-$C_{20}$ alkinyl, $Sn(R_a)_3$, $B(OR_a)_2$ and $Si(R_a)_3$, B and B', which may be the same or different, may be C, N or P, preferably C or N, and o and p may be 0 or 1.

Diamines wherein one of Z and Z' represents $Sn(R_a)_3$, halogen or boronic acid esters are preferred if the compounds of formula (1) obtained from such diamines are used as reagents for the synthesis of oligomers or homo- or copolymers comprising moieties or repeating units or for the synthesis of compounds which comprise monovalent or divalent groups derived from compounds of formula (1), which will be described in more detail hereinafter.

$R_a$ and $R_b$, which may be the same or different, are selected from $C_1$-$C_{20}$ hydrocarbyl groups or $C_1$-$C_{20}$ heterohydrocarbyl groups, preferably from $C_1$-$C_{12}$ alkyl groups.

Preferred substituents Z" and Z"' are hydrogen, halogen or $C_1$-$C_{12}$ alkyl groups.

As is immediately apparent to the skilled person, compounds in which B and B' are C and Z" and Z"' are H are derivatives of 1,2-diaminobenzene. Replacing one C by N yields derivatives of 2,3-diaminopyridine whereas compounds with both B and B' yield derivatives of 2,3-diamino-1,4-pyrazine.

Z and Z" respectively Z' and Z"' may also form together a ring.

Just by way of example a number of suitable diamines of the above general formula are given below:

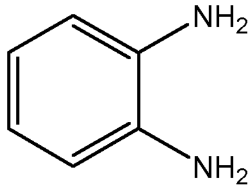

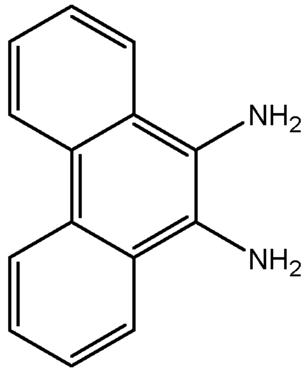

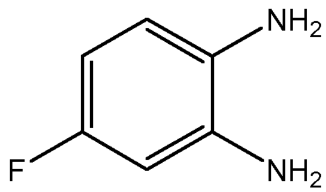

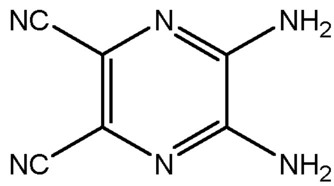

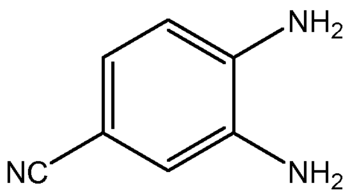

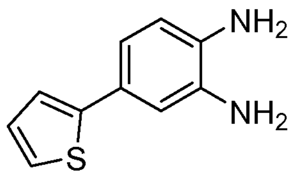

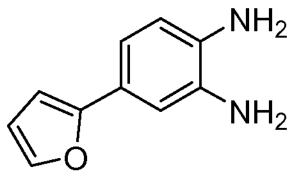

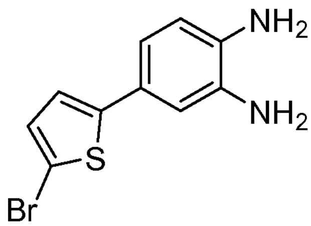

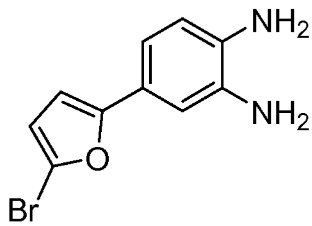

Further suitable diamines, just given by way of example, are the following, which comprise a five membered heteroaryl ring instead of a six membered ring to which the amino groups are attached.

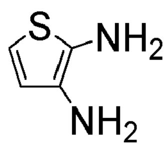

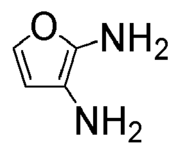

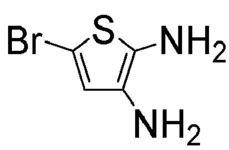

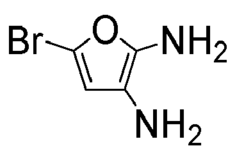

Phenanthrene-9,10-diamine and diaminophenazine and derivatives may also be mentioned here as suitable diamines for the manufacture of the compounds in accordance with the present invention.

The foregoing representative examples of suitable diamines show that the skilled person can select from a great variety of different diamines to obtain specific compounds of formulae (1) and (2).

Another embodiment of the present invention are oligomers or homo- or copolymers comprising moieties or repeating units of formula (12)

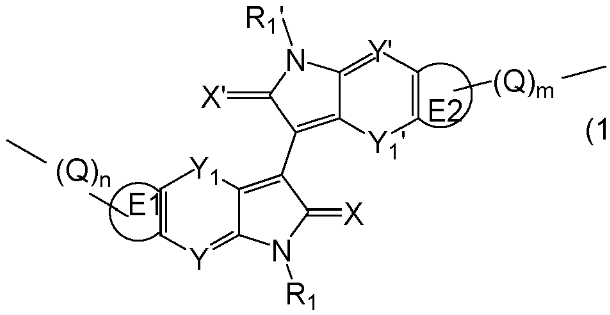

(12)

wherein X, X', Y, Y', $Y_1$, $Y_1$', $R_1$, $R_1$', E1 and E2 have the meanings as defined above,
Q, which may be the same or different at each occurrence, represents a
$C_1$-$C_8$ alkylene group or a substituted or unsubstituted five or six membered aromatic or heteroaromatic ring, and
m and n, independent of another, are 0 or 1.

The term moiety, as used herein, for the purposes of the present invention shall mean that the oligomer or polymer comprises a respective group as repeating unit or as part of the repeating unit. Thus, in the latter case, the repeating unit may comprise additional structural elements or groups besides those depicted in formula (12).

The term oligomers, when used herein, denotes products having a maximum of ten repeating units or moieties of formula (12) whereas the term polymers is intended to denote products having more than ten units of formula (12).

Oligomers or polymers comprising only units of formula (12) may be obtained by polycondensation using tetramines instead of diamines in the reaction scheme given above for the manufacture of compounds of formula (1).

The principle of this reaction type is shown below for a specific tetramine and a homopolymerization by polycondensation, but it is readily apparent to the skilled person that the reactants may be modified to obtain specific desired polymers

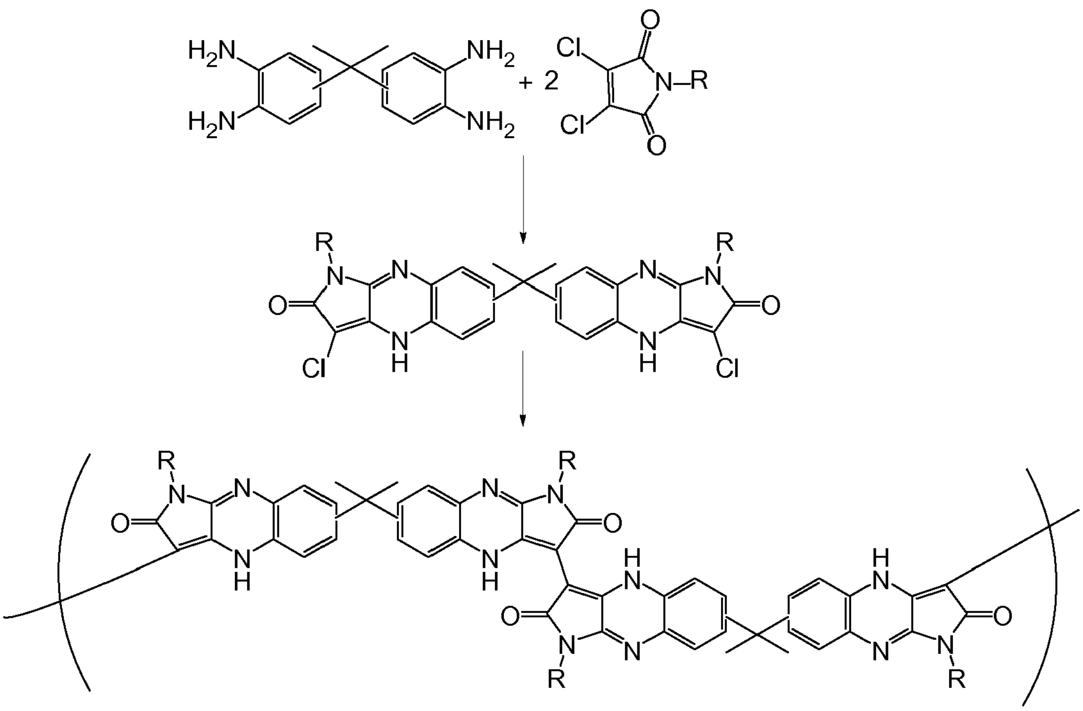

By way of example, some suitable tetramines are shown below, but it is readily apparent to the skilled person that suitable tetramines can be chosen from a broad variety of compounds so that the compounds shown below are not limiting the scope of the tetramines which may be used but only serve as examples to show the general principle.

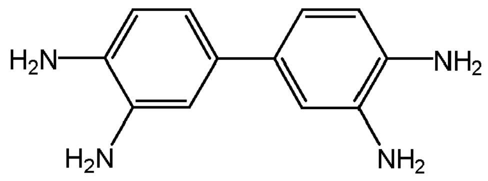
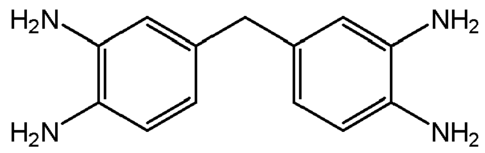
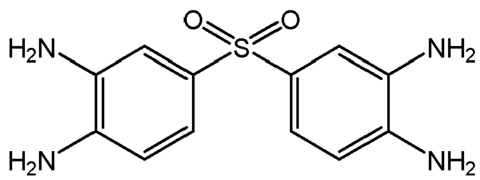
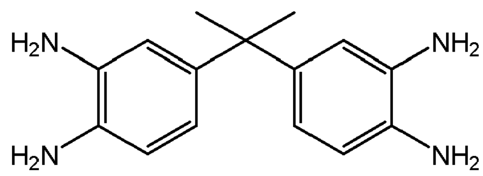

The polymerization by polycondensation avoids the use of heavy metal catalysts and the need to remove catalyst residues after the reaction, which is economically advantageous and environmentally preferable.

As already indicated above, respective oligomers and polymers of formula (12) may also be obtained from compounds of formula (1) bearing substituents which enable coupling reactions. Suitable substituents of this type are halides, organotin compounds or boronic acid esters, which may be used in coupling reactions like the Stille or Suzuki coupling or the Heck and Buchwald Hartwig reaction to obtain oligomers or polymers if two compounds of formula (1) with respective substituents are reacted with each other, i.e. a compound of formula (1) having a trialkyl tin substituent may be reacted with a compound of formula (1) having a halide substituent, or other difunctional compounds not conforming to formula (1) or (2) may be used as co-reactants to obtain copolymers having a desired combination of different repeating units.

Exemplary compounds of formula (1) having respective functionalities are shown below and the skilled person will select the appropriate reactants dependent on the specific application and the structure of the desired polymer

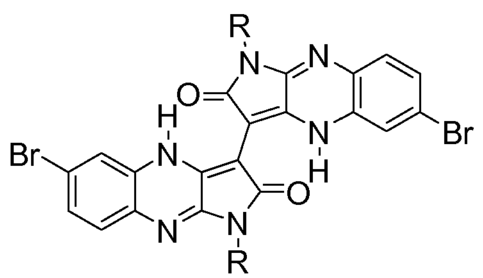
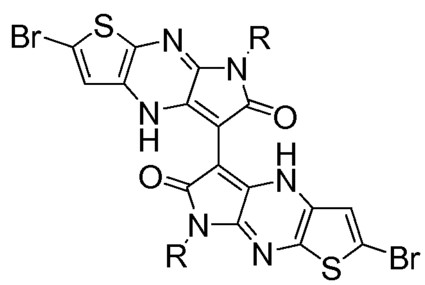
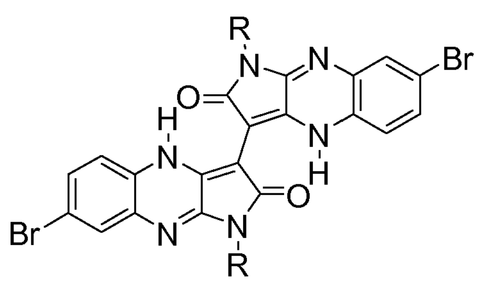
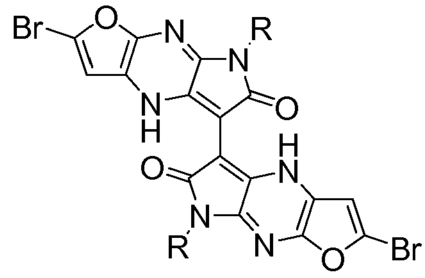

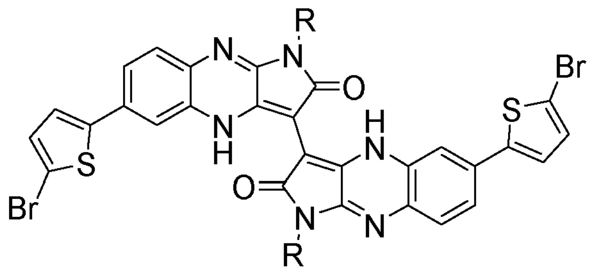

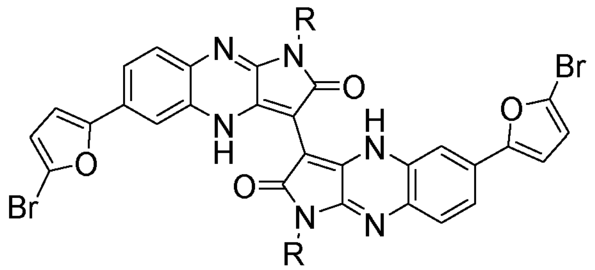

Bromo compounds, e.g. those shown above, may also be used as starting materials to obtain the respective triorgano tin derivatives or boronic acid ester derivatives and a triorgano tin derivative or a boronic acid ester derivative may be reacted with a bromine derivative in a Stille, Suzuki, Heck or Buchwald Hartwig coupling reaction, usually using Pd compounds as catalysts.

These reactions have been broadly described in the literature and the skilled person will select the appropriate reactants and reaction conditions based on his professional knowledge and the specific case to carry out the coupling and to obtain the desired oligomers or polymers.

If starting materials with two organotin or boronic acid ester groups of formulae (1) or (2) are reacted with starting materials of formulae (1) or (2) having two bromine substituents, and the reaction conditions and the molar ratios of the reactants are chosen appropriately, oligomers and polymers with moieties or repeating units of formula (12) can be obtained.

If one of the starting materials is selected from compounds not conforming with formula (1) or (2) co-oligomers or copolymers are obtained comprising at least two different moieties respectively repeating units. This way, the properties of the respective products may be fine tuned to achieve the best result for a specific application.

The common structural feature of all these oligomers and polymers is the presence of moieties or repeating units of formula (12) which are derived from compounds of formulae (1) or (2).

A preferred group of co-oligomers or copolymers in accordance with the present invention are obtained by coupling compounds of formulae (1) or (2) with compounds having electron acceptor properties as in many cases the compounds of formulae (1) and (2) have electron donating properties.

This kind of reaction is shown below for a specific preferred group of compounds of formula (2) but it is apparent to the skilled person that the any compound of formulae (1) or (2) may be used in order to obtain a specific structure.

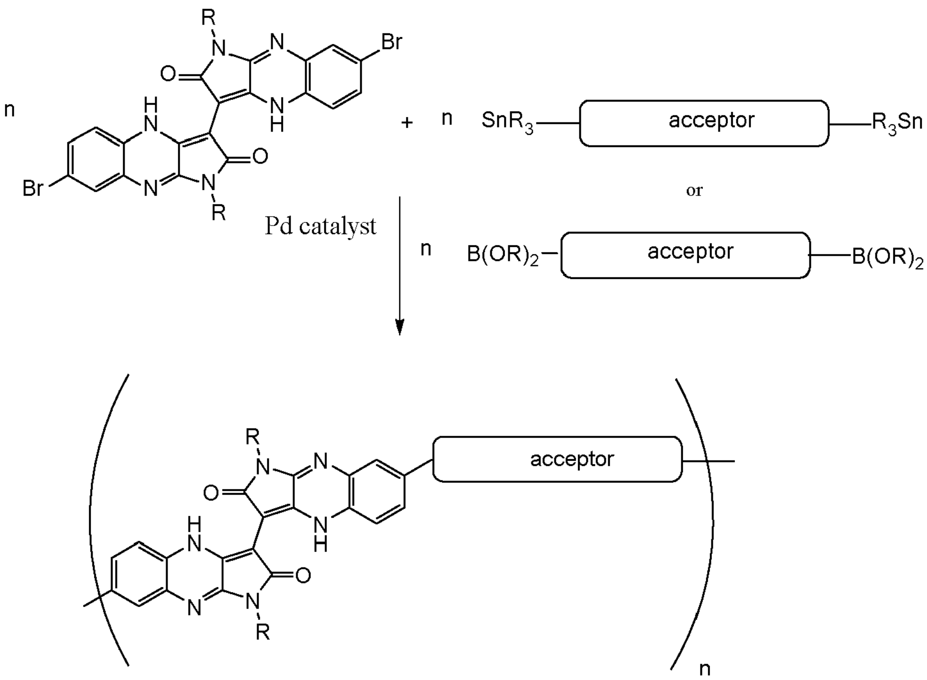


Preferred electron acceptor compounds suitable as comonomers for copolymerization in accordance with the above general scheme are rylene diimides, fullerene derivatives or diketopyrrolopyrrole derivatives, to name only a few examples.

An exemplary respective copolymer obtained by reaction of a compound of formula (2) and a naphthalene diimide compound as a representative of the group of rylene diimides is shown below

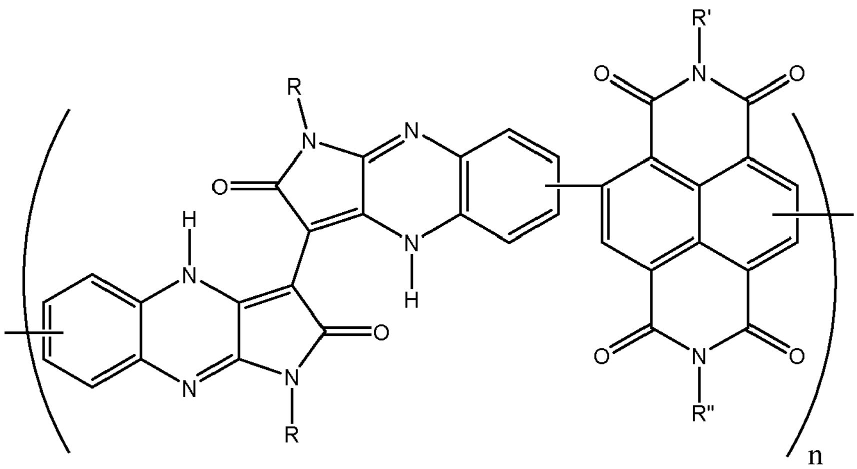

The molecular weight of the homo- or copolymers in accordance with the present invention is not subject to limitations, i.e. can vary over a broad range. Solubility of the polymers may depend on the degree of polymerization and thus it may be desirable to limit the molecular weight to increase the solubility, which is advantageous if the polymers are processed for final use by solution processing methods.

Still another embodiment of the present invention relates to compounds of formulae (13) to (15)

A - D (13)

A - D' A (14)

D - A' - D (15)

wherein

A is a monovalent electron acceptor group, preferably based on a rylene diimide, a fullerene derivative or a diketopyrrolopyrrole, particularly preferred based on a rylene diimide,

D is a monovalent electron donor group based on a compound in accordance with the present invention as specified above,

D' is a divalent electron donor group based on a compound in accordance with the present invention as specified above and

A' is a divalent electron acceptor group, preferably based on a rylene diimide, a fullerene derivative or a diketopyrrolopyrrole, particularly preferred based on a rylene diimide.

Rylene diimides may be characterized by the general formula

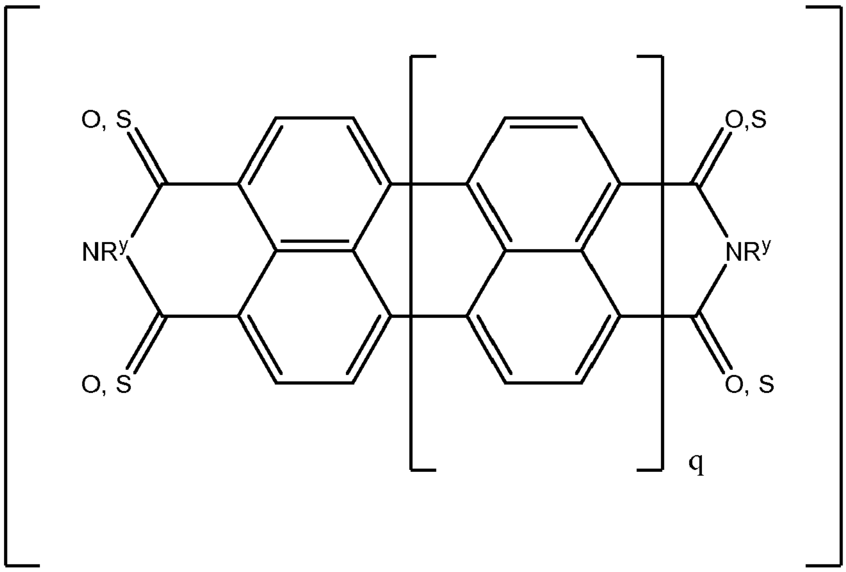

(15)

wherein Ry may be hydrogen, a $C_1$-$C_{20}$ hydrocarbyl a $C_1$-$C_{20}$ heterohydrocarbyl group and q is an integer of from 0 to 3. The aromatic rings may be substituted or unsubstituted and may also form annealed ring systems with other aromatic or heteroaromatic rings.

Zhan et al., Adv. Mater. 2011, 268-284 provide an overview on rylene diimides which have been found suitable for applications in organic electronics.

Rylene diimides for the purpose of the present invention share the common feature of two six membered dicarboxylic acid imide rings fused to terminal naphthalene units. The three simplest rylene diimide systems are naphthalene-1,8:4,5 tetracarboxylic acid diimides (hereinafter referred to as NDIs), perylene-3,4:9,10-tetracarboxylic acid diimides (hereinafter referred to as PDIs and terylene-3,4:11.12-tetracarboxylic acid diimides reproduced below

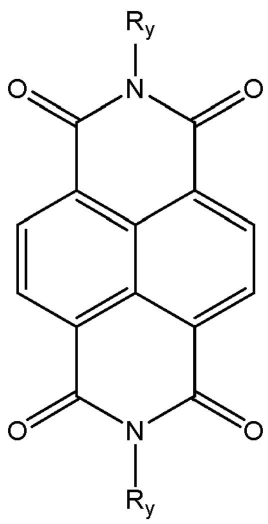

NDI

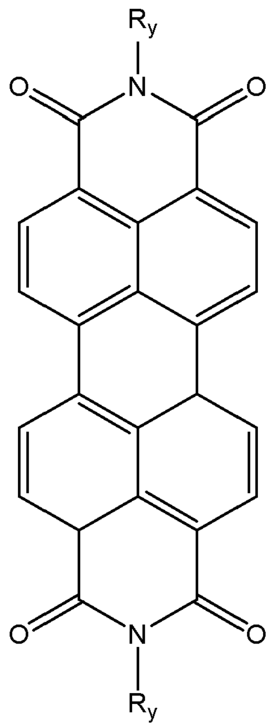

PDI

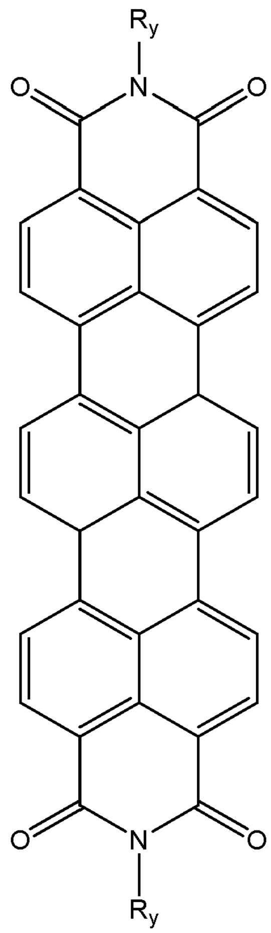

Terylene diimide

where the aromatic rings may be substituted or unsubstituted and where one or more of the oxygen atoms may be replaced by sulfur.

Suitable rylene diimides are known and commercially available or have been described in the literature or may be obtained by processes known to the skilled person so that no further details are necessary here.

Naphthalene diimide derivatives and perylene diimide derivatives and in particular NDIs are preferred electron acceptors in the compounds of formulae (13) to (15).

Fullerene derivatives for use in organic electronic devices have also been broadly described in the literature.

The term fullerene generally denotes molecules composed entirely of carbon in the form of hollow spheres, ellipsoids or tubes. They are similar in structure to graphite, which is composed of stacked graphene sheets of linked hexagonal rings. Fullerenes may also comprise pentagonal and sometimes heptagonal rings. In mathematical terms fullerenes could be characterized as trivalent convex polyhedron with pentagonal and hexagonal faces.

The most common fullerene is C60-fullerene, but C70, C72, C76, C84 and C100-fullerenes are also known.

For use in organic electronics, fullerenes are generally derivatized and one derivative, commonly referred to as PCBM has been thoroughly investigated as electron acceptor material in organic electronic devices. PCBM denotes the fullerene derivative [6,6]-phenyl-C61 butyric acid methyl ester.

Other fullerene derivatives have been described in the literature and are known to the skilled person so that no more details are necessary here.

Diketopyrrolopyrrole derivatives, frequently referred to as DKPP derivatives represent another preferred group of electron acceptors which might be used to obtain the compounds of formulae (13) to (15) in accordance with the present invention.

DKPP compounds share the common structural element

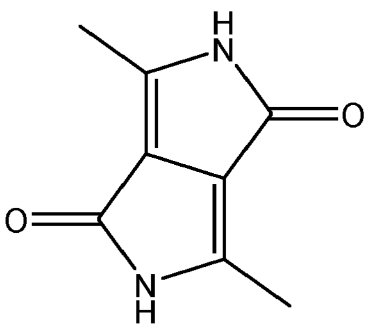

the basic compound of the family being 2,5-dihydropyrrolo[3,4-c]-pyrrole-1,4-dione (both substituents being hydrogen in this case).

The oxygen atoms in the DKPP basic structure may be replaced partly or fully by sulfur atoms, thus yielding the respective thio- or dithioderivatives.

Suitable DKPP based compounds for use in organic electronic devices have been described in the literature and are known to the skilled person so that no further details need to be given here.

The compounds of formulae (13) to (15) may be obtained according to processes and coupling reactions known to the skilled person and described in the literature. Only by way of example Stille-, Suzuki-, Heck- and Buchwald-Hartwig coupling reactions may be mentioned here.

As preferred examples of compounds of formula (13) to (15) the following two examples of trimeric compounds based on a compound of formula (1) and a NDI are shown

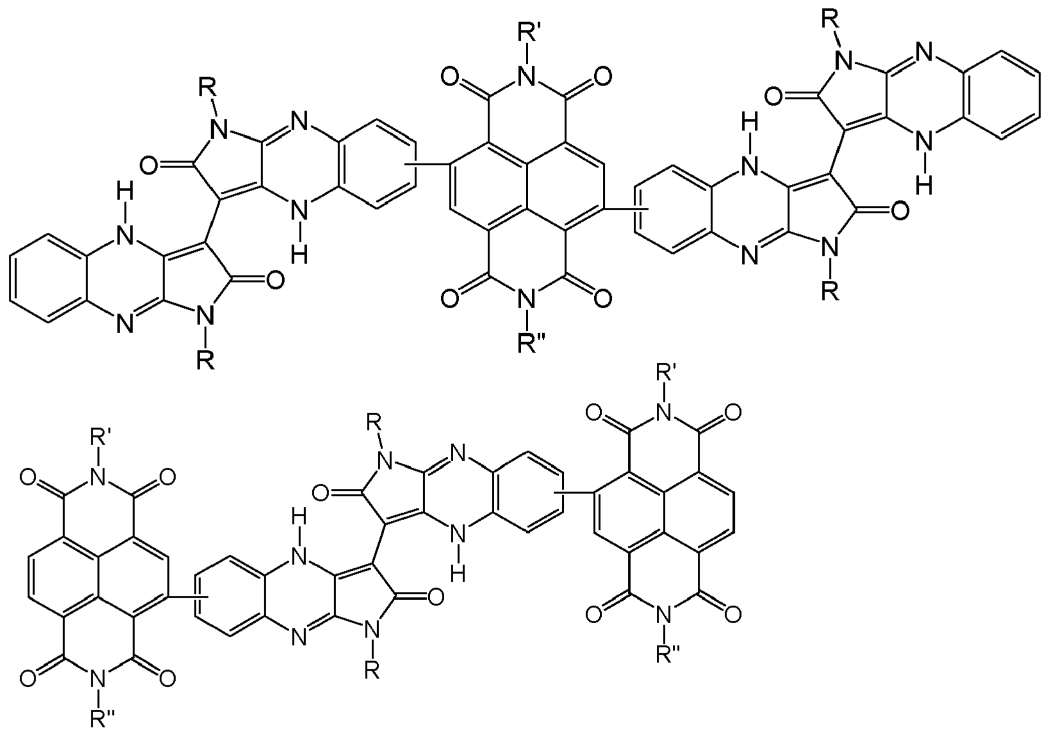

The first compound corresponds to formula (14) whereas the second compound corresponds to formula (15).

Another embodiment of the present invention relates to compositions of matter comprising a compound in accordance with the present invention, or an oligomer, homo-or copolymer in accordance with the present invention as electron donor and an electron acceptor compound, preferably selected from the group consisting of fullerene derivatives, rylene diimides and diketopyrrolopyrrole derivatives or mixtures thereof.

For use in organic electronic devices the compounds in accordance with the present invention or the oligomers, homo-or copolymers may be formed into semiconducting films by a variety of methods known to the skilled person and described in the literature. Vapor deposition methods as well as solution-based methods may be mentioned here. Such semiconducting films constitute another embodiment of the present invention.

Preferably the semi-conducting films are obtained in the form of self-assembled monolayers (SAM), i.e. molecular assemblies formed spontaneously on surfaces by adsorption or chemisorption of head groups onto a substrate from either the vapor or liquid phase.

By selecting the suitable type of head group the properties of such self assembled monolayers can be adjusted in accordance with the needs of the specific application.

The compounds in accordance with the present invention, represented e.g. by formula (1), (2), and (13) to (15) or the oligomers, homo-or copolymers comprising moieties or repeating units of formula (12) or the compositions of matter in accordance with the present invention may be used for forming one or more layers of organic electronic devices or may constitute a component of such layers in organic electronic devices.

The compounds in accordance with the present invention or the oligomers, homo-or copolymers in accordance with the present invention may be ambipolar in nature or may show electron conductivities and hole conductivities differing by several orders of magnitude, which makes them particularly suitable for use in electron transport layers or hole transport layers of organic electronic devices.

Especially for the use in organic field effect transistors (OFET) such difference in the conductivity for different charge carriers can be advantageous.

Three essential components of field-effect transistors are the source, the drain and the gate. Field-effect transistors usually operate as a capacitor and are frequently composed of two plates. One plate works as a conducting channel between two ohmic contacts which are called the source and the drain. The other plate works to control the charge induced into the channel and is called the gate. The direction of the movement of the carriers in the channel is from the source to the drain.

Field effect transistors are unipolar transistors as they rely on single carrier-type operation and therefore a difference in conductivity for different charge carriers may be advantageous when the compounds or compositions of the present invention are used in field effect transistors.

The compounds, or oligomers, homo-or copolymers or compositions of matter in accordance with the present invention may also be used as semiconducting films in organic photovoltaic devices.

The compounds in accordance with the present invention are particularly suitable as efficient organic semiconductors, either in the form of small molecules or as building blocks in polymeric architectures.

In many cases the compounds in accordance with the present invention are efficient p-type organic semiconductors showing a good charge transport and a good stability under ambient conditions.

By appropriate substitution of the conjugated cores with electron withdrawing groups the HOMO and LUMO of the compounds can be tuned in accordance with the specific needs of the individual application. By grafting various chains the solubility and the $\pi$-stacking can be promoted and the organic semiconducting properties generally improved.

The mobility of the compounds in accordance with the present invention is improved compared to compounds known from the prior art, which can also be seen from the following examples.

Due to the good solubility of the compounds in accordance with the present invention, thin films can be prepared by e.g. spin coating out of different solvents with good reproducibility.

High performance organic semiconductors can advantageously be used for flexible electronic applications like e.g. display backplanes or RFID tags. Other examples are so called E-readers.

The mobility required for such devices has to reach certain minimum values and the compounds or the compositions in accordance with the present invention show good mobilities and thus can form the basis for the development of respective organic electronic devices.

Furthermore, it has been found that the compounds in accordance with the present invention can be homopolymerized by condensation reactions without the need to use expensive catalysts, which have to be recovered through costly steps and without the need of the elimination of dangerous side products. Accordingly, the compounds in accordance with the present invention are particularly suitable for the formation of polymeric organic semiconductors which can be obtained by environmentally friendly processes.

**Example 1 Synthesis of compound of formula (3)**

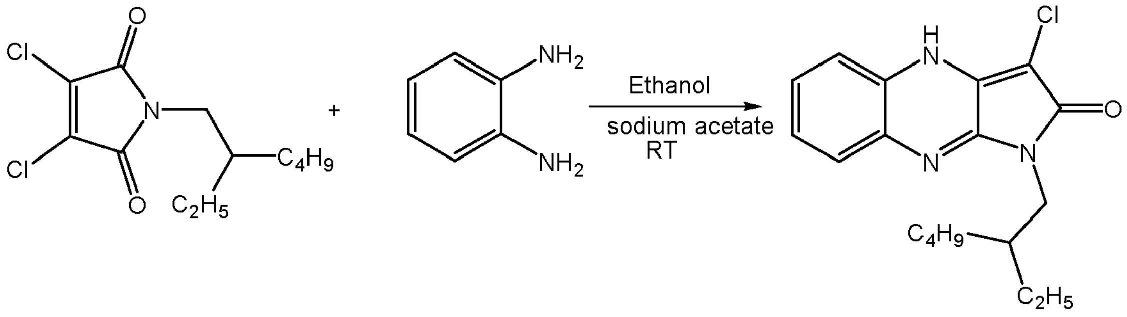


In a round bottom flask (250ml) 3,4-dichloro-1-(2-ethylhexyl)-1H-pyrrole-2,5-dione (14g, 50.4mmol) was dissolved under argon in 150ml of absolute ethanol. Sodium acetate and 1,2-diaminobenzene were added. The mixture was stirred overnight at room temperature before being poured into 500ml of water. 14g of a yellow solid were recovered by filtration. The product was used without further purification in the following step.

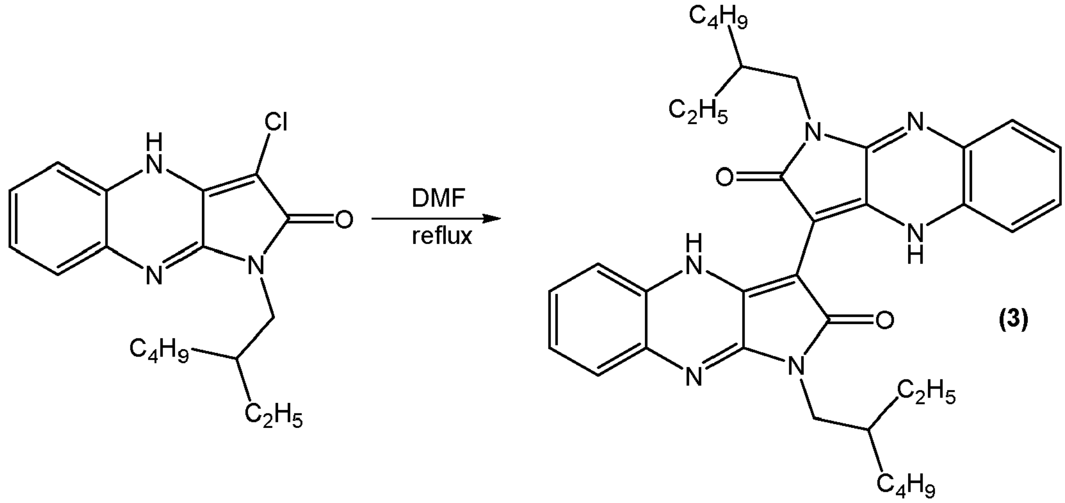


(3)

In a round bottom flask (100ml) under argon, 3-chloro-1-(2-ethylhexyl)-1H-pyrrolo[2,3-b]quinoxalin-2(3H)-one (0.5g, 1.5mmol) was added to 20ml of DMF. After refluxing overnight and cooling to room temperature the red solid was filtered and washed with water and ethanol to give 0.29g (60%) of pure material (3). This product was only slightly soluble in organic solvents.

**Example 2 - Synthesis of compound of formula (4)**

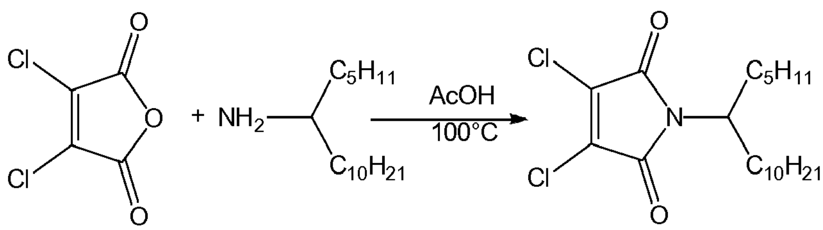


In a dried 3 neck round bottom flask (11) 2,3-dichloromaleic anhydride (24g, 0.144mol)) was dissolved under argon atmosphere in 400ml of acetic acid. 6-hexadecylamine (36g, 0.149mol) was added and the reaction medium was refluxed during 3 hours. The solvent was removed under vacuum and the product was dissolved in 500ml of ethyl acetate. After washing with 200ml of HCL 0.1N, 200ml of NaOH 0.1N and 200ml of saturated NaCl solution, the organic layer was dried over $MgSO_4$. After removal of the solvent, 39g of the respective dichloromaleimide (3,4-dichloro-1-(hexadecan-6-yl)-1H-pyrrole-2,5-dione) was recovered with a yield of 69%. The product was used without further purification

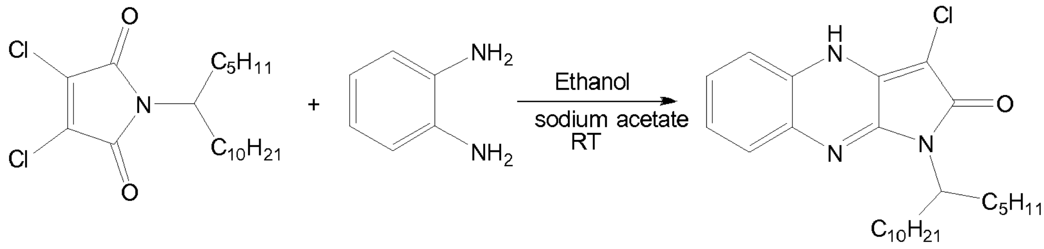


In a round bottom flask (250ml) 3,4-dichloro-1-(hexadecan-6-yl)-1H-pyrrole-2,5-dione (10g, 25.6 mmol) was dissolved under argon in 150ml of absolute ethanol. Sodium acetate (2.5g, 30.7mmol) and 1,2-diaminobenzene (2.77g, 26.6 mmol) were added. The mixture was stirred overnight at room temperature. Ethanol was removed by distillation under vacuum at 40°C and the crude product was purified by flash chromatography onto silica gel starting from hexane/ethylacetate 90/10 and finishing with hexane/ethylacetate 70/30 as the eluents. 9g of pure product was obtained as yellow wax with a yield of 78%.

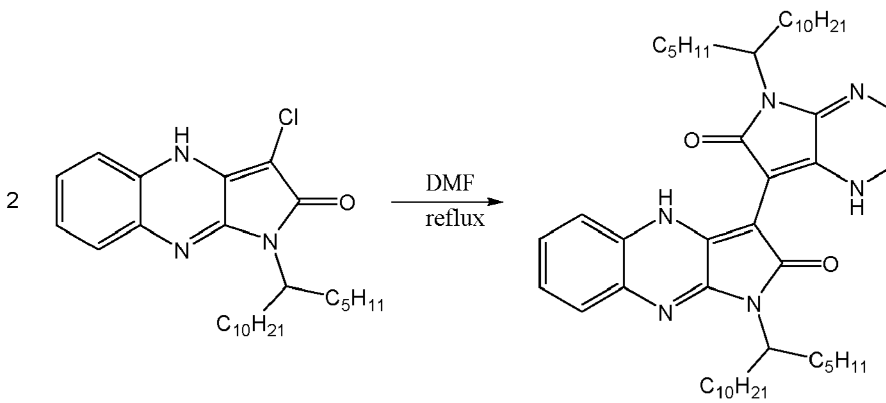


**(4)**

In a round bottom flask (250ml) under argon, 3-chloro-1-(hexadecan-6-yl)-1 H-pyrrolo[2,3-b]quinoxalin-2(4H)-one (6g, 13.5mmol) was added to 150ml of DMF. After refluxing overnight the reaction medium was cooled to room temperature. A red solid was filtered and washed with 3X50ml of ethanol to give 3.5g (63%) of pure product (4). This material did not require any further purification and was soluble in organic solvents.

**Example 3 - Synthesis of compound of formula (5)**

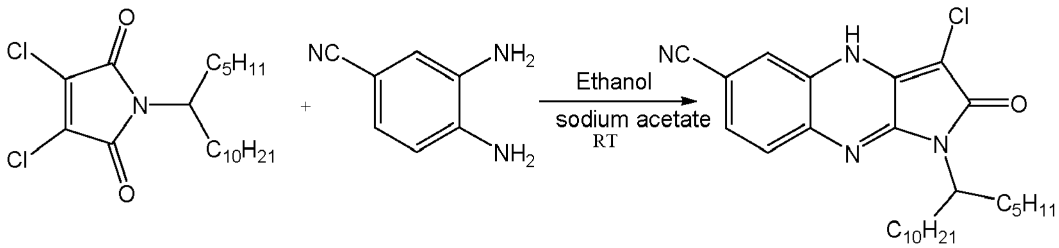


In a round bottom flask (250ml) 3,4-dichloro-1-(hexadecan-6-yl)-1H-pyrrole-2,5-dione (10g, 25.6 mmol) was dissolved under argon in 150ml of absolute ethanol. Sodium acetate (2.5g, 30.7mmol) and 3,4-diaminobenzonitrile (3.4g, 25.6 mmol) were added. The mixture was stirred overnight at room temperature. Ethanol was removed by distillation under vacuum at 40°C and the crude product was purified by flash chromatography onto silica gel using hexane/methylene-chloride/ethylacetate 60/35/5 as the eluent. 2.5g of pure product were obtained as yellow wax with a yield of 21 %. When conditions were made harsher by heating to reflux the final product (5) was obtained (yield 40%).

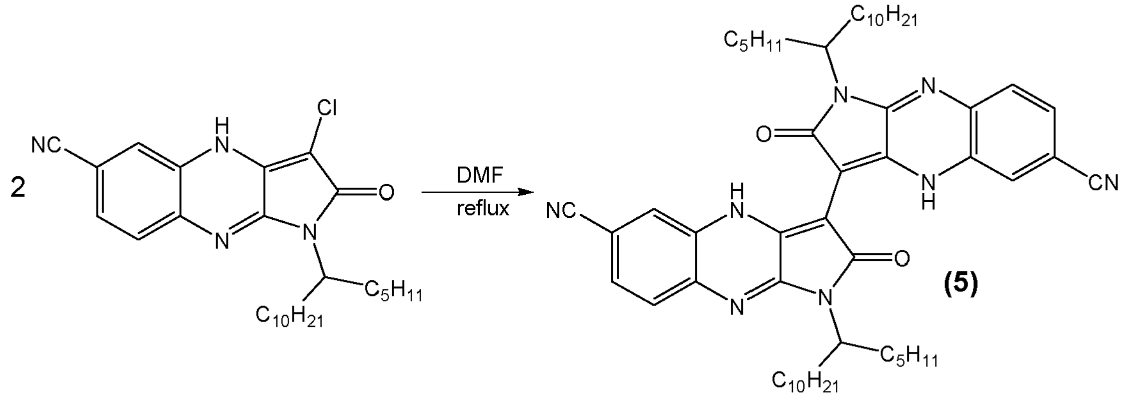


**(5)**

In a round bottom flask (100ml) under argon, 3-chloro-1-(hexadecan-6-yl)-2-oxo-2,4-dihydro-1H-pyrrolo[2,3-b] quinoxaline-6-carbonitrile (2.5g, 5.3mmol) was added to 50ml of DMF. After refluxing overnight the reaction medium was cooled to room temperature. A red solid was filtered and washed with 3X50ml of methanol to give 0.8g (34%) of pure material (5). This product did not require any further purification and was soluble in organic solvents

**Example 4 - Synthesis of compound of formula (9)**

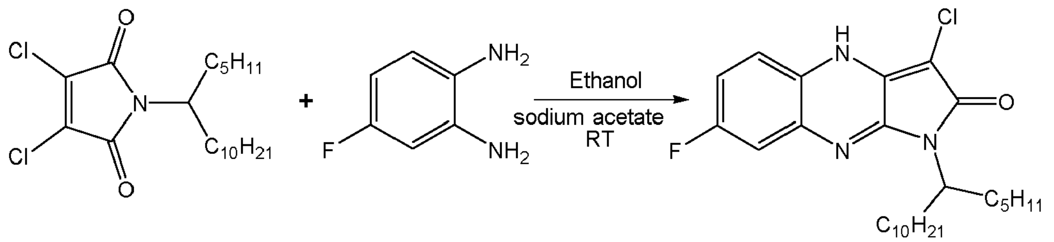


In a round bottom flask (250ml) 3,4-dichloro-1-(hexadecan-6-yl)-1H-pyrrole-2,5-dione (16g, 41 mmol) was dissolved under argon in 150ml of absolute ethanol. Sodium acetate (3.9g, 47.6mmol) and 4-fluoro-1,2-diaminobenzene (5.0g, 39.7mmol) were added. The mixture was stirred overnight at room temperature. Ethanol was removed by distillation under vacuum at 40°C and crude product was purified by flash chromatography onto silica gel starting from hexane/methylene chloride 75/25 and finishing with hexane/methylenechloride/ethylacetate 70/25/5 as the eluents. 7g of pure product were obtained as yellow wax with a yield of 38%. Thin layer chromatography monitoring revealed that some isomers besides red final dihydrogenoisoindigo (9) were formed.

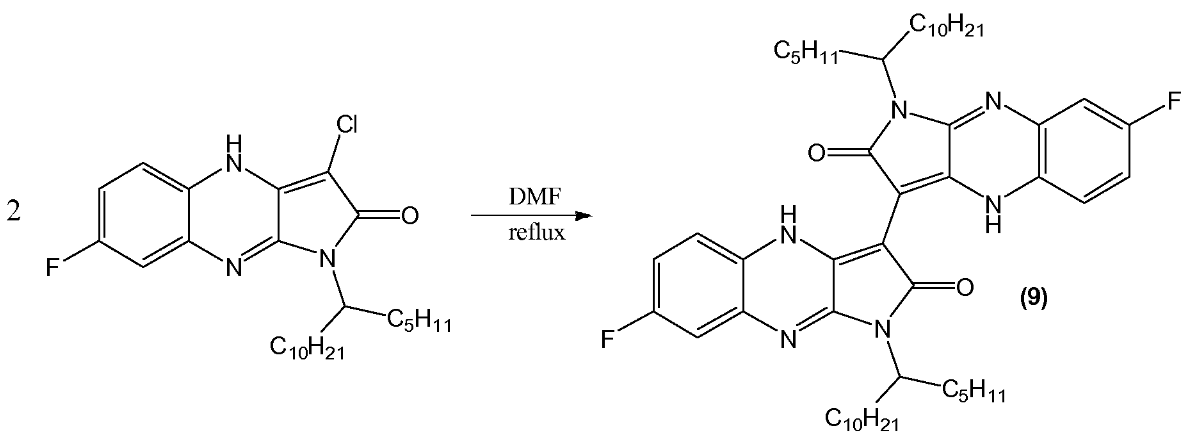


**(9)**

In a round bottom flask (250ml) under argon, 3-chloro-6-fluoro-1-(hexadecan-6-yl)-1H-pyrrolo[2,3-b]quinoxalin-2(3H)-one (6.9g, 15mmol) was added to 150ml of DMF. After refluxing overnight the reaction medium was cooled to room temperature. A red solid was filtered and washed with 3X50ml of methanol to give 3.9g (61 %) of pure material (9). This product did not require any further purification and was soluble in organic solvents.

**Example 5 - Synthesis of compound of formula (7)**

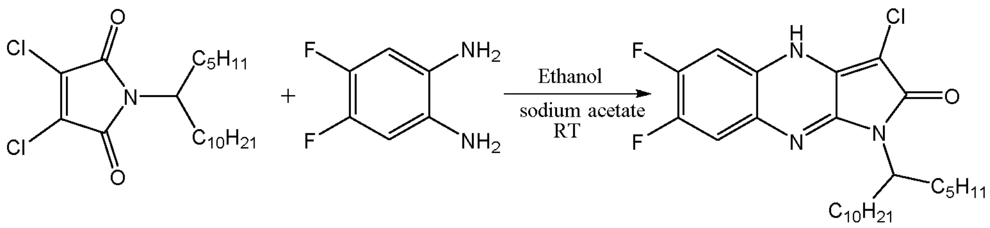


In a round bottom flask (250ml) 3,4-dichloro-1-(hexadecan-6-yl)-1H-pyrrole-2,5-dione (14.4g, 37mmol) was dissolved under argon in 150ml of absolute ethanol. Sodium acetate (2.96g, 37mmol) and 4,5-difluoro-1,2-diaminobenzene (4.32g, 30mmol) were added. The mixture was stirred during 20h at room temperature. Monitoring by thin layer chromatography showed that the reaction was incomplete and that the final dihydrogenoisoindigo (7) product was already formed at this stage. Ethanol was removed by distillation under vacuum at 40°C and crude product was extracted with ethylacetate. After washing with water, drying the organic phase and removal of the solvent, 19g of crude product were obtained. Purification by flash chromatography onto 600g silica gel starting from hexane/methylene chloride 75/25 and finishing with hexane/methylenechloride/ethylacetate 70/25/5 as the eluents gave 0.8g of the red dihydrogenoisoindigo (7) and 8g of the expected product (55%). The latter was a yellow wax which tended to crystallize after several days.

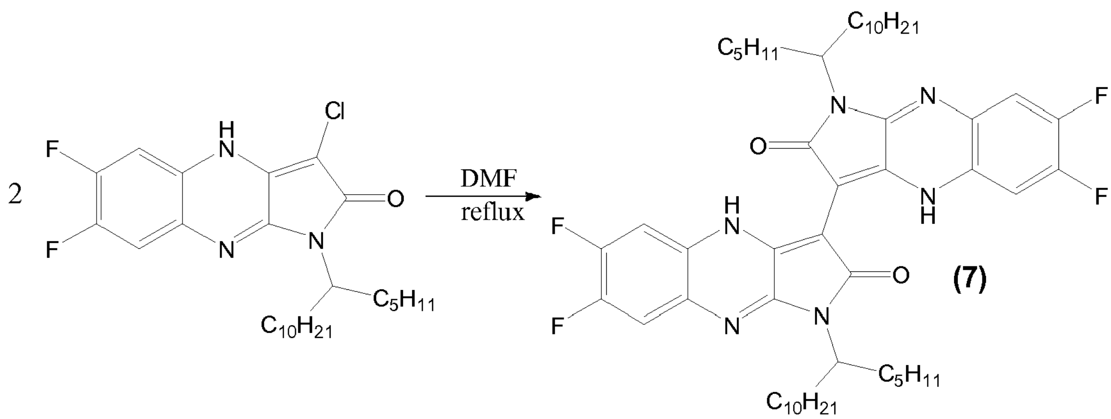

(7)

In a round bottom flask (250ml) under argon, 3-chloro-6,7-difluoro-1-(hexadecan-6-yl)-1H-pyrrolo[2,3-b]quinoxalin-2(4H)-one (7.7g, 16mmol) were added to 100ml of DMF. After refluxing overnight the reaction medium was cooled to room temperature. A red solid was filtered and washed with 3X50ml of methanol to give 4.0g (56%) of pure material (7). This product did not require any further purification and was soluble in organic solvents.

**Example 6 - Synthesis of compound of formula (8)**

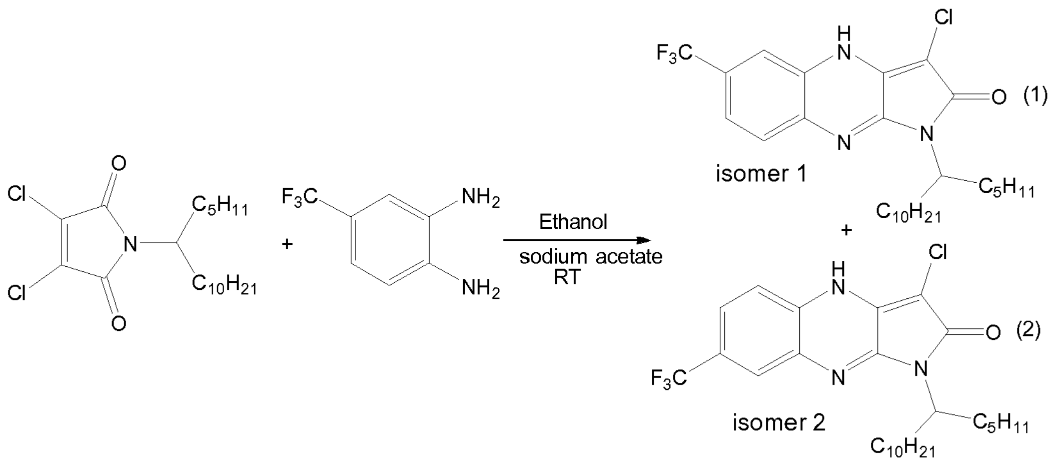

(1) isomer 1 + (2) isomer 2

In a round bottom flask (250ml) 3,4-dichloro-1-(hexadecan-6-yl)-1H-pyrrole-2,5-dione (14.4g, 37mmol) was dissolved under argon in 150ml of absolute ethanol. Sodium acetate (2.96g, 37mmol) and 4-trifluoromethyl-1,2-diaminobenzene (5.28g, 29.5mmol) were added. The mixture was stirred for 24h at room temperature. Monitoring by thin layer chromatography showed that reaction was incomplete and not selective as 2 isomers were formed. Ethanol was removed by distillation under vacuum at 40°C and crude product was extracted with ethylacetate. After washing with water, drying the organic phase and removal of the solvent, crude product was obtained. 4,6g of isomer 1 and 2.0g of isomer 2 (combined yield was 40%) were obtained after purification by flash chromatography onto silica gel starting from hexane/methylene chloride 75/25 and finishing with hexane/methylenechloride/ethylacetate 75/15/10 as the eluents. The products were yellow waxes which tended to crystallize after a few days.

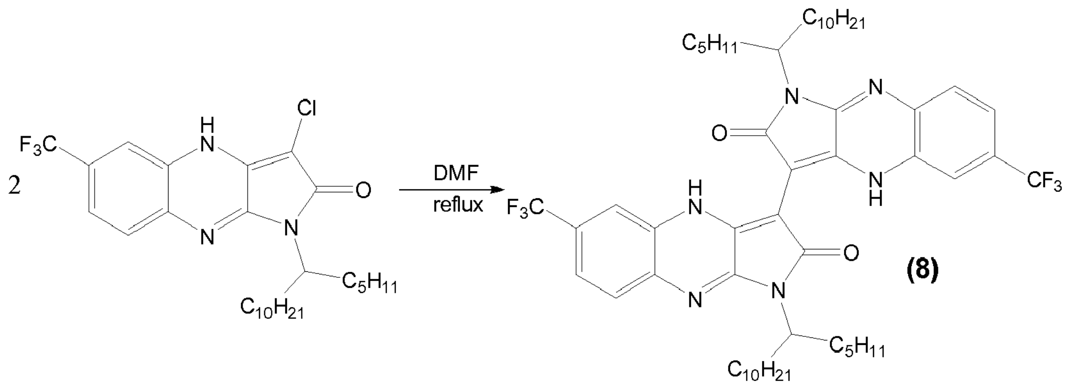


(8)

In a round bottom flask (100ml) under argon, 3-chloro-1-(hexadecan-6-yl)-6-(trifluoromethyl)-1H-pyrrolo[2,3-b]quinoxalin-2(4H)-one (2.0g, 3.9mmol) was added to 40ml of DMF. After refluxing overnight the reaction medium was cooled to room temperature. A red solid was filtered and washed with 3X50ml of methanol to give 0.9g (48%) of pure material (8). This product did not require any further purification and was soluble in organic solvents.

The properties of the compounds synthesized in Examples 1 to 6 above are summarized in Table 1.

Table 1

| Compound of Example | LUMO (eV) | HOMO (eV) | Melting point °C | μ (mobility) $cm^2/Vs$ |
|---|---|---|---|---|
| 1 | | | 254 | |
| 2 | -2.6 | -5.05 | 111 | 0.15 |
| 3 | -2.9 | -5.3 | 267 | 0.06 |
| 4 | | -5.1 | 173 | 0.14 |
| 5 | | -5.2 | 154 | |
| 6 | | -5.3 | 245 | |

The HOMO and LUMO levels of the organic molecules used in the process of the present invention are determined from cyclic voltammetry measurements in solution as follows:

The measurements are performed at room temperature, under inert atmosphere, with a conventional three-electrode configuration, the solution being outgassed before use with a stream of argon for 5-10 min. The three-electrode cell may consist e.g of a glassy carbon disk as working electrode, a Pt wire or a Pt rod as a counter electrode and a Pt wire or a carbon rod as pseudo-reference electrode. Ferrocene is used as an internal reference. Other cell configurations may also be used. The solvents used for the determination of the HOMO and LUMO levels are respectively anhydrous dichloromethane and anhydrous tetrahydrofuran, the supporting electrolyte is 0.1 M tetrabutylammonium hexafluorophosphate and the host concentrations are 2 - 0.5 millimolar. The scan rate is fixed to 100 mv/s.

The HOMO levels ($E_{HOMO}$) of the organic molecules used in the process of the present invention are calculated from the measured half wave potential of their first oxidation wave ($E_{1\ ox\ 1/2}$) using the following equation:

$$E_{HOMO} - (-4.8) = -[E_{1\ ox\ 1/2} - E_{ox\ 1/2}(Fc/Fc^{+})]$$

wherein the ferrocene HOMO level value has been taken equal to -4.8 eV below the vacuum level according to Pommerehene and al. Adv. Mater. 7(6), 551-554 (1995) and wherein $E_{ox\ 1/2}(Fc/Fc^{+})$ corresponds to the measured

half wave potential of the ferrocene oxidation wave. For irreversible systems, $E_{pa\ 1}$ peak potential value of the first oxidation wave is used instead of the half wave potential $E_{1ox\ 1/2}$.

The LUMO levels ($E_{LUMO}$) of the organic molecules used in the process of the present invention are calculated from the measured half wave potential of their first reduction wave ($E_{1ox\ 1/2}$) using the following equation:

$$E_{LUMO} - (-4.8) = -[E_{1\ red\ 1/2} - E_{ox\ 1/2}(Fc/Fc^+)]$$

wherein the ferrocene HOMO level value has been taken equal to -4.8 eV below the vacuum level according to Pommerehene et al. Adv. Mater. 7(6), 551-554 (1995) and wherein $E_{ox\ 1/2}(Fc/Fc^+)$ corresponds to the measured half wave potential of the ferrocene oxidation wave. For irreversible systems, $E_{pc\ 1}$ peak potential value of the first reduction wave is used instead of the half wave potential $E_{1\ red\ ½}$.

If no reduction wave is observed in tetrahydrofuran, the LUMO level is calculated from the HOMO level deduced from cyclic voltammetry as hereabove and from the optical energy bandgap $E_{opt\ g}$ measured from the wavelength of the absorption edge $\lambda_{abs.\ edge}$ using the following equations:

$$E_{opt\ g}\ (eV) = 1240/\lambda_{abs.\ edge}\ (nm); \quad E_{LUMO}\ (eV) = E_{HOMO}\ (eV) + E_{opt\ g}\ (eV).$$

If there is a risk of an overlap of the oxidation waves of ferrocene and the compound under evaluation, decamethylferrocene can be introduced into the medium as a reference. The equations used for the calculation of the HOMO and LUMO levels are then:

$$E_{HOMO} - (-4.8 + (E_{ox\ 1/2}(Fc/Fc^+) - E_{ox\ 1/2}(Me_{10}Fc/Me_{10}Fc^+))) = -[E_{1\ ox\ 1/2} - E_{ox\ 1/2}(Me_{10}Fc/Me_{10}Fc^+)]$$

$$E_{LUMO} - (-4.8 + (E_{ox\ 1/2}(Fc/Fc^+) - E_{ox\ 1/2}(Me_{10}Fc/Me_{10}Fc^+))) = -[E_{1\ red\ 1/2} - E_{ox\ 1/2}(Me_{10}Fc/Me_{10}Fc^+)]$$

where $E_{ox\ 1/2}(Fc/Fc^+)$-$E_{ox\ 1/2}(_{Me10}Fc/_{Me10}Fc^+)$ are values reported in table 2 of D. Astruc et al., Can.J.Chem.84, 288-299 (2006).

For determining mobility as provided in Table 1, organic thin film transistor devices (OTFT's) with a Top Gate Bottom contact geometry were tested in air using a standard cascade probe station in conjunction with an Agilent B1501 semiconductor parameters analyzer. The Agilent system calculated the saturation mobility according to the equation

$$\mu_{sat} = \frac{2L}{W}\frac{1}{C_i}\left(\frac{\partial I_D^{0.5}}{\partial V_G}\right)^2_{V_D}$$

where L is the transistor length, W is the transistor width, $C_i$ is the dielectric capacitance per unit area, $I_D$ is the drain-source current and $V_G$ is the gate-source voltage. $V_D$ is the drain-source voltage and was set at - 100V.

The general structure of the devices used for measurement is shown in Fig. 1 wherein reference numeral 1 designates the Gate (Al), reference numeral 2 designates the dielectric layer, reference numerals 3 and 6 depict self assembled monolayers, reference numeral 4 stands for the drain (Au), reference numeral 5 stands for a buffer layer, reference numeral 7 depicts the source (Au), reference numeral 8 represents the organic semiconductor and reference numeral 9 depicts the bottom (glass).

## Claims

1. Compounds of general formula (1)

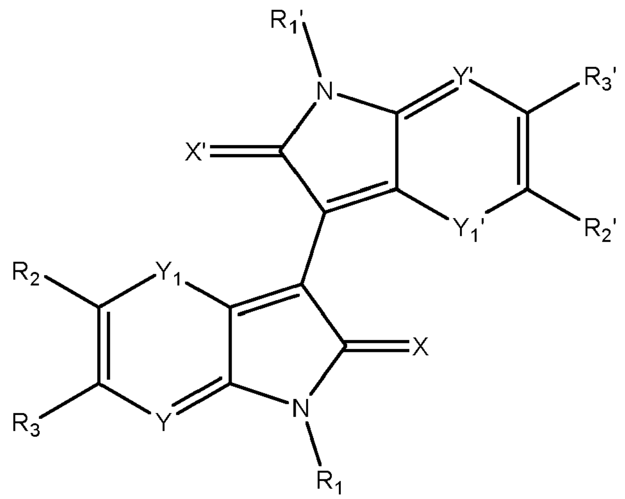

(1)

wherein X and X', independent of each other, represent O or S,
Y and Y', independent of each other represent N, or P,
$Y_1$ and $Y_1$', independent of each other, represent NH, O, -S- or PH $R_1$ and $R_{1'}$, independent of each other represent hydrogen, a $C_1$-$C_{30}$ hydrocarbyl or a $C_1$-$C_{30}$-heterohydrocarbyl group,
$R_2$, $R_3$, $R_2$' and $R_3$', independent of each other represent hydrogen, halogen, a $C_1$-$C_{20}$ hydrocarbyl or a $C_1$-$C_{20}$ heterohydrocarbyl group or $R_2$ and $R_3$ respectively $R_2$' and $R_3$' form together a substituted or unsubstituted five or six membered carbocyclic or heterocyclic, aromatic or heteroaromatic ring, which ring may be part of a fused ring system.

**2.** Compounds in accordance with claim 1 represented by formula (2)

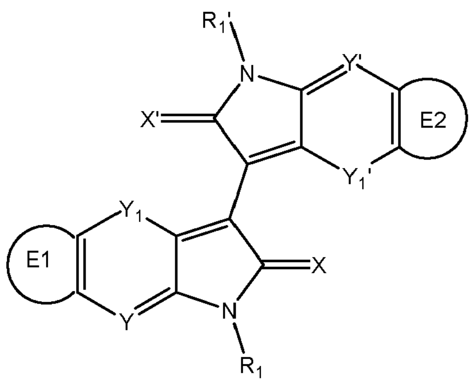

(2)

wherein E1 and E2 represent a substituted or unsubstituted five or six membered aromatic or heteroaromatic ring.

**3.** Compounds in accordance with claim 2, wherein the five or six membered heteroaromatic ring is selected from

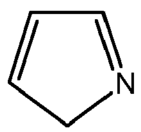 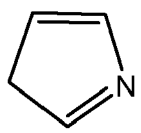 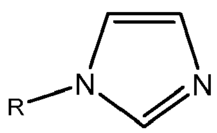 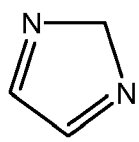 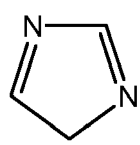

2*H*pyrrole | 3*H*pyrrole | 1-substituted 1 *H*-imidazole | 2H-imidazole | 4 *H*-imidazole

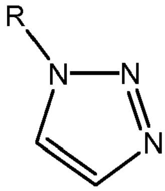
1-substituted-1 *H*-1,2,3-triazole

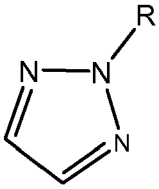
2-substituted-2H-1,2,3-triazole

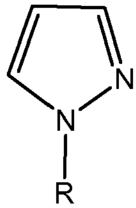
1-substituted-1 *H* -pyrazole

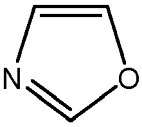
oxazole

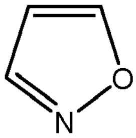
isoxazole

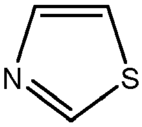
thiazole

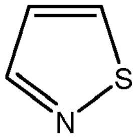
isothiazole

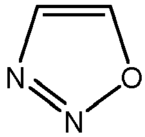
1,2,3-oxadiazole

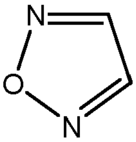
1,2,5-oxadiazole

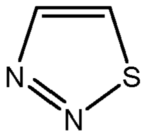
1,2,3-thiadiazole

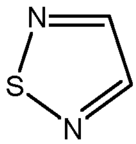
1,2,5-thiadiazole

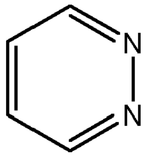
pyridazine

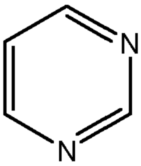
pyrimidine

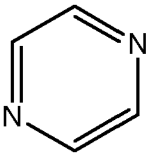
pyrazine

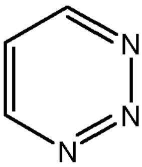
1,2,3-triazine

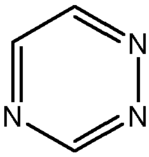
1,2,4-triazine

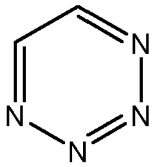
1,2,3,4-tetrazine

**4.** Compounds in accordance with any of claims 1 to 3 wherein $R_1$ and $R_1$' are identical and X and X' are identical.

**5.** Compounds in accordance with any of claims 1 to 4 wherein Y is identical to Y' and $Y_1$ is identical to $Y_1$'.

**6.** Compounds in accordance with claim 5 wherein Y and Y' are N and $Y_1$ and $Y_1$' are NH.

**7.** Compounds in accordance with any of claims 1 to 6 represented by the following formulae (3) to (11)

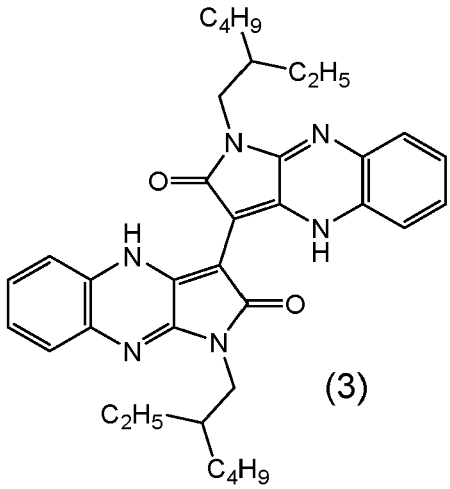

(3)

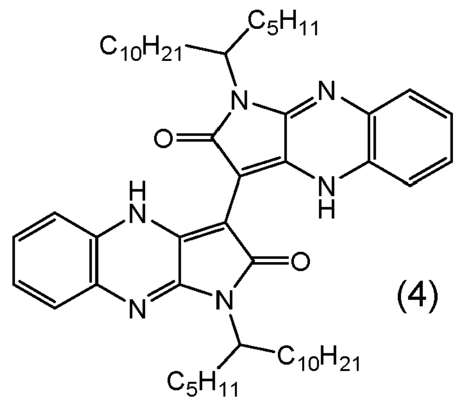

(4)

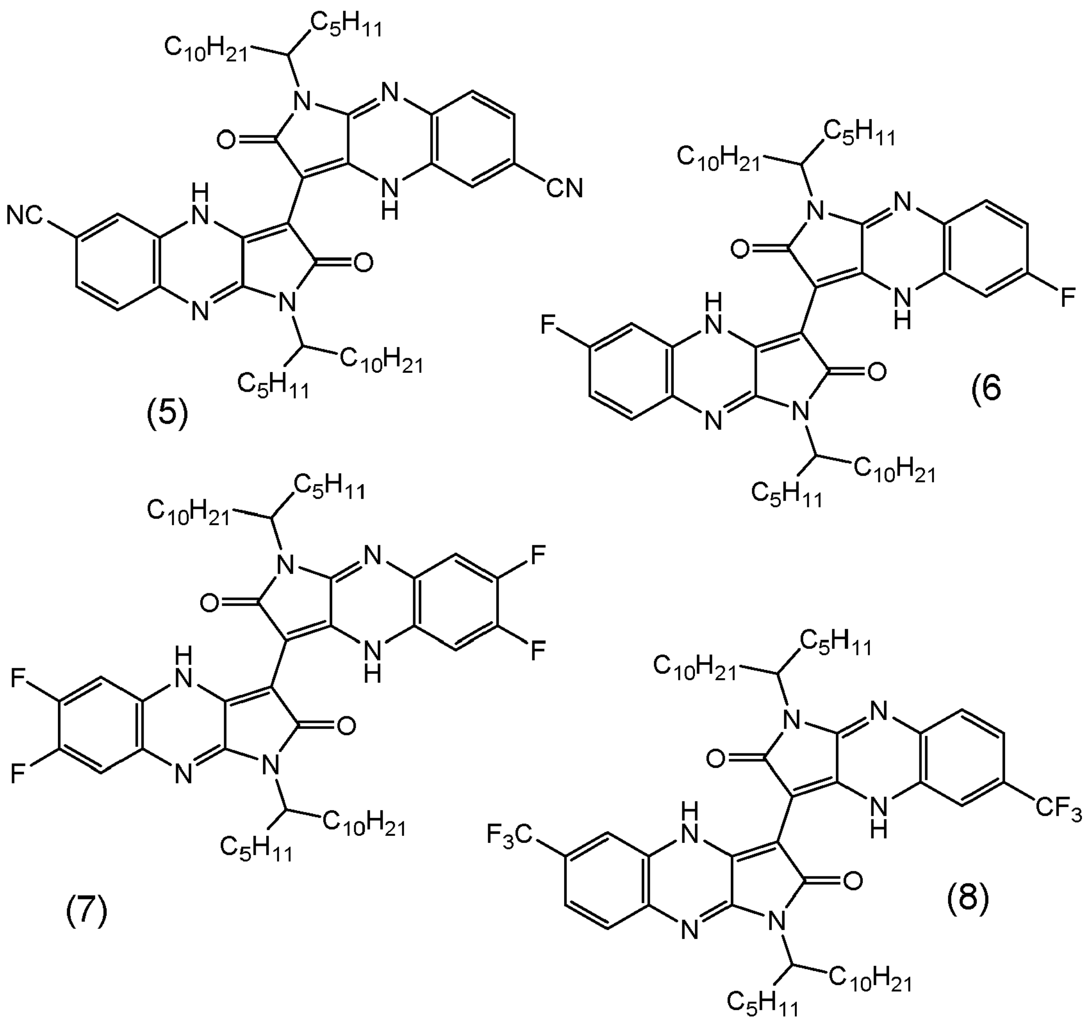

(5)

(6

(7)

(8)

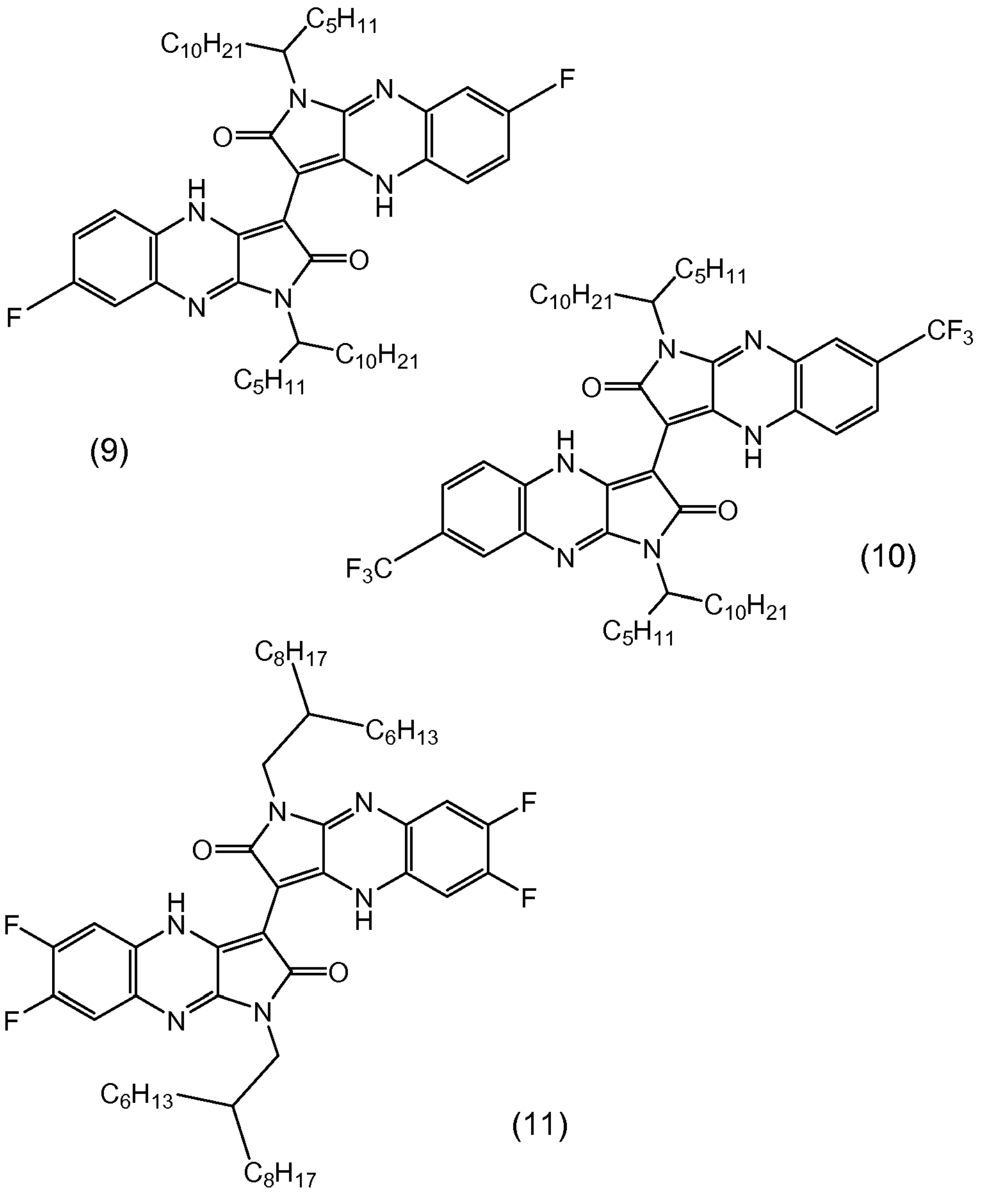

(9)

(10)

(11)

8. Oligomers or homo- or copolymers comprising moieties or repeating units of formula (12)

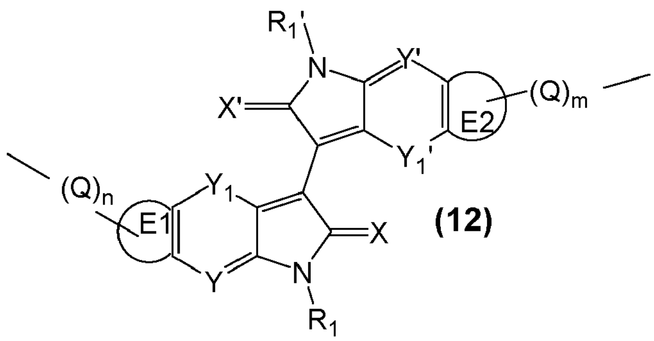

**(12)**

wherein X, X', Y, Y', $Y_1$, $Y_1$', $R_1$, $R_1$',E1 and E2 have the meanings as defined in claims 1 to 7, Q, which may be the same or different at each occurrence, represents a C1-C8-alkylene group or a substituted or unsubstituted five or six membered aromatic or heteroaromatic ring, and

m and n, independent of another, are 0 or 1..

**9.** Compounds of formulae (13) to (15)

A-D (13)

A-D'A (14)

D-A'-D (15)

wherein
A is a monovalent electron acceptor group, preferably based on a rylene diimide, a fullerene derivative or a diketopyrrolopyrrole, particularly preferred based on a rylene diimide,
D is a monovalent electron donor group based a compound in accordance with any of claims 1 to 7,
D' is a divalent electron donor group based on a compound in accordance with any of claims 1 to 7 and
A' is a divalent electron acceptor group, preferably based on a rylene diimide, a fullerene derivative or a diketopyrrolopyrrole, particularly preferred based on a rylene diimide.

**10.** Compositions of matter comprising a compound in accordance with any of claims 1 to 7 and 9 or an oligomer, homo- or copolymer in accordance with claim 8 as electron donor and an electron acceptor compound, preferably selected from the group consisting of fullerene derivatives, rylene diimides and diketopyrrolopyrrole derivatives or mixtures thereof.

**11.** Semiconducting film comprising a compound as defined in claims 1 to 7 and 9 or an oligomer, homo- or copolymer in accordance with claim 8 or a composition in accordance with claim 10 or mixtures thereof.

**12.** Organic photovoltaic device comprising a semiconducting film in accordance with claim 11.

**13.** Organic light emitting device comprising a semiconducting film in accordance with claim 11 as hole transport layer or electron transport layer.

**14.** Organic field effect transistor comprising a semiconducting layer based on a semiconducting film in accordance with claim 11.

**15.** Use of the compounds in accordance with claims 1 to 7 or 9 or of oligomers, homo- or copolymers in accordance with claim 8 or compositions of matter in accordance with claim 10 or mixtures thereof for transporting holes or electrons in a layer of an organic electronic device.

**Figure 1**

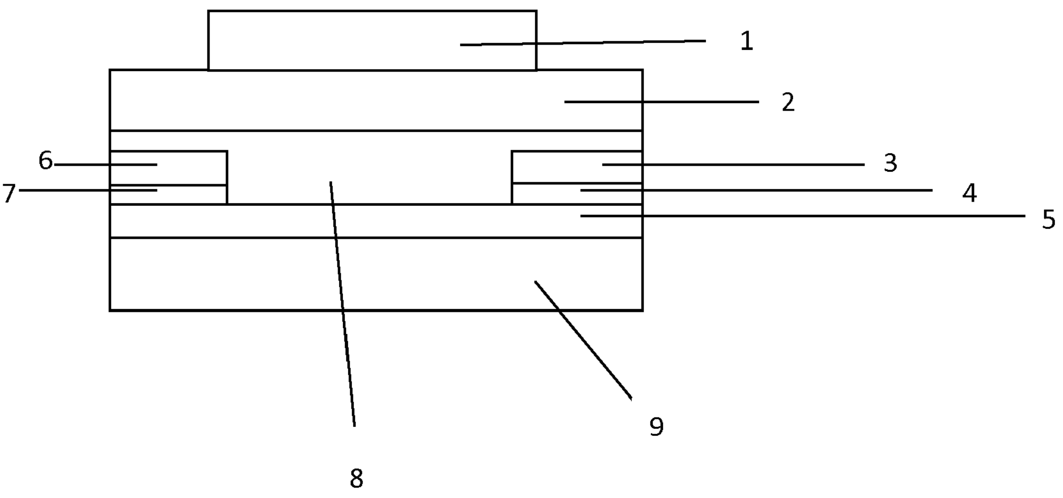

Europäisches Patentamt
European Patent Office
Office européen des brevets

# EUROPEAN SEARCH REPORT

Application Number
EP 13 16 9825

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FR 1 410 733 A (BASF AG) 10 September 1965 (1965-09-10) * claim 1; examples * | 1,2,4-6 | INV. C07D487/04 H01L51/00 |
| X | HISAO NISHI ET AL: NIPPON KAGAKU KAISHI, no. 1, 1977, pages 146-149, XP055080252, ISSN: 0369-4577, DOI: 10.1246/nikkashi.1977.146 | 1,2,4-6 | |
| Y | * abstract; figure 2; example 4a to 4f * | 1-15 | |
| Y | TING LEI ET AL: "Influence of Alkyl Chain Branching Positions on the Hole Mobilities of Polymer Thin-Film Transistors", ADVANCED MATERIALS, vol. 24, no. 48, 18 December 2012 (2012-12-18), pages 6457-6461, XP055080433, ISSN: 0935-9648, DOI: 10.1002/adma.201202689 * the whole document * | 1-15 | |
| | | | TECHNICAL FIELDS SEARCHED (IPC) |
| | | | C07D H01L |

The present search report has been drawn up for all claims

3

| Place of search | Date of completion of the search | Examiner |
|---|---|---|
| Munich | 24 September 2013 | Miniejew, Catherine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 16 9825

This annex lists the patent family members relating to the patent documents cited in the above-mentioned European search report. The members are as contained in the European Patent Office EDP file on
The European Patent Office is in no way liable for these particulars which are merely given for the purpose of information.

24-09-2013

| Patent document cited in search report | Publication date | Patent family member(s) | Publication date |
|---|---|---|---|
| FR 1410733 A | 10-09-1965 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 1196205

### Non-patent literature cited in the description


- **REYNOLDS et al.** *Macromolecules,* 2010, vol. 43, 8348-8352
- **REYNOLDS et al.** *Organic Letters,* 2010, vol. 12 (4), 660-663
- **REYNOLDS et al.** *Macromolecules,* 2011, vol. 44, 6303-6310
- **PEI et al.** *Adv. Mater.,* 2012
- **REICHENEDER et al.** *Angew. Chem.,* 1969, vol. 81 (19), 742
- **ZHAN et al.** *Adv. Mater.,* 2011, 268-284
- **POMMEREHENE.** *Adv. Mater.,* 1995, vol. 7 (6), 551-554
- **POMMEREHENE et al.** *Adv. Mater.,* 1995, vol. 7 (6), 551-554
- **D. ASTRUC et al.** *Can.J.Chem.,* 2006, vol. 84, 288-299